# EUROPEAN PATENT APPLICATION

(11) **EP 4 101 470 A1**
(43) Date of publication of application: **14.12.2022**
(21) Application number: 21750079.2
(22) Date of filing: 21.01.2021
(51) Int. Cl.: A61K 47/54, A61K 47/65, A61K 38/07, A61K 38/08, A61P 35/00, A23L 33/18, A23L 33/12

(54) **UNSATURATED FATTY ACID-CONJUGATED CP2C-TARGETING PEPTIDE-BASED ANTICANCER AGENT**

(30) Priority: 05.02.2020 KR 20200013901
(71) Applicant: Industry-University Cooperation Foundation Hanyang University, Seoul 04763 (KR)
(72) Inventor: KIM, Chul Geun, Seoul 03981 (KR)
(74) Representative: dompatent von Kreisler Selting Werner - Partnerschaft von Patent- und Rechtsanwälten mbB
(86) International application number: PCT/KR2021/000847
(87) International publication number: WO 2021/157916

(57) **Abstract**

The present invention relates to an unsaturated fatty acid-conjugated CP2c-targeting peptide-based agent. In the present invention, it was confirmed that by linking an unsaturated fatty acid to ACP52C, which is a CP2c-targeting peptide lead substance exhibiting an effect as a general-purpose anticancer agent, *in vivo* stability was improved without affecting the anticancer effect. Further, cancer cell-specific anticancer efficacy was confirmed in various cancer cells and normal cells.

## Description

### [Technical Field]

The present invention relates to a CP2c-targeting peptide-based anticancer agent.

### [Background Art]

Although many protein/peptide anticancer agents and small molecule compound-based anticancer agents have been developed and used to date, such anticancer agents do not selectively act only on cancer cells *in vivo* and induce serious side effects that also affect normal cells, and their efficacy is insignificant due to the emergence of drug-resistant cancer cells. In addition, since many protein/peptide anticancer agents under development have short half-lives *in vivo,* various studies have been conducted to overcome this disadvantage. Although efforts have been competitively made worldwide to develop anticancer agents, an anticancer agent free from problems with *in vivo* stability, safety and drug resistance has not yet been developed. Therefore, if an anticancer agent which secures stability capable of being maintained *in vivo* for a long period of time, can selectively remove only various types of cancer cells, and also acts on drug-resistant cancer cells is developed, a high added value will be able to be brought worldwide.

### [Disclosure]

### [Technical Problem]

An object of the present invention is to provide a CP2c-targeting peptide-based anticancer agent which secures stability capable of being maintained *in vivo* for a long period of time, can selectively remove only various types of cancer cells, and also acts on drug-resistant cancer cells.

### [Technical Solution]

A CP2c-targeting peptide according to the present invention refers to a peptide that induces cancer cell-specific cell death by binding to a transcription factor CP2c to suppress the formation of a CP2c transcription factor complex (CP2c homotetramer and CP2c/CP2b/PIAS1 heterohexamer). As the CP2c-targeting peptide, Tyr-Pro-Gln-Arg (SEQ ID NO: 1) consisting only of amino acids essential for an anticancer effect, corresponds to the smallest sized peptide. Therefore, a peptide that essentially includes the four amino acids and exhibits an anticancer effect by interacting with the CP2c protein may be used as the CP2c-targeting peptide according to the present invention.

The CP2c-targeting peptide according to the present invention may be a peptide consisting of 4 to 20 amino acids including the amino acid sequence of SEQ ID NO: 1. Preferably, the CP2c peptide according to the present invention may be a peptide (ACP52) including 6 amino acids (6 aa) 'NYPQRP(Asn-Try-Pro-Gln-Arg-Pro, SEQ ID NO: 2)'.

The CP2c-targeting peptide according to the present invention may be used by binding to a cell-penetrating peptide (CPP) for enhancing cell permeation activity.

For example, the CP2c-targeting peptide according to the present invention may be a peptide including internalizing RGD (iRGD) which is a peptide consisting of 9 amino acids (9aa) '[Cys-Arg-Gly-Asp-Lys-Gly-Pro-Asp-Cys (CRGDKGPDC), SEQ ID NO: 3]' as a peptide consisting of Asn-Try-Pro-Gln-Arg- Pro (NYPQRP) and a cell-penetrating peptide (CPP). 'ACP52C', which is an example of the CP2c-targeting peptide according to the present invention, is a peptide in which iRGD binds to the C-terminal Pro of ACP52 and NH₂ (amide group) binds to the C-terminus of iRGD.

In a previous study, a CP2c-targeting peptide-fatty acid conjugate binding to a saturated fatty acid palmitoyl acid was prepared for the purpose of securing the *in vivo* stability of the CP2c-targeting peptide conventionally developed by the present applicant, and it was expected that when a fatty acid is allowed to bind to a CP2c-targeting peptide, the *in vivo* stability is increased by binding to albumin in blood to provide a high therapeutic effect. However, its actual effect was inferior to that of the case where the CP2c-targeting peptide was used alone, which was determined that palmitoyl acids aggregate with each other as a saturated fatty acid to form a micelle structure, and thus the CP2c-targeting peptide was unable to bind to albumin, and a cell-penetrating peptide iRGD also entered into the micelle to suppress the delivery to cancer cells. It was determined that unsaturated fatty acids that cannot form a micelle can be used as a way to overcome such problems, and as a result of analyzing characteristics as a cancer therapeutic agent in various cancer cell lines and Hep3B xenograft mouse models by synthesizing complexes in which all-cis-docosa-4,7,10,13,16,19-hexa-enoic acid (DHA) was bound to ACP52 and ACP52C, respectively, excellent effects were confirmed, thereby completing the present invention.

The unsaturated fatty acid may be a C₁₂ to C₂₂ fatty acid but is not limited thereto. In an exemplary embodiment of the present invention, the unsaturated fatty acid may be a C₂₂ unsaturated fatty acid, for example, all-cis-docosa-4,7,10,13,16,19-hexa-enoic acid (DHA).

The CP2c-targeting peptide-fatty acid conjugate according to the invention may include a CP2c-targeting peptide (for example, ACP52) that provides CP2c-targeting and a CPP (for example, iRGD) and/or a linker peptide linking them between fatty acids. As the linker peptide, a peptide consisting of amino acids known in the art or a combination thereof can be used without limitation. Specifically, the linker peptide may include glycine (Gly, G), for example, Gn (here, n is an integer from 1 to 6).

In another exemplary embodiment of the present invention, the linker peptide may consist of an amino acid sequence represented by GₙKGₘ (here, n and m are each independently an integer from 0 to 6). For example, when n or m is 0, lysine (Lys, K) may be located at the N-terminus or C-terminus of the linker peptide, and when n and m are not 0, lysine may be located between glycines. Lysine (Lys, K) included in the linker peptide is included for the conjugation of the fatty acid and peptide. Specifically, a terminal functional group (-HN₂) of lysine enables the binding of additional amino acids. Thus, the linker peptide of the present invention may additionally include an amino acid sequence represented by EGLFG, which is a target sequence of glutamic acid (Glu, E) that binds to a functional group of lysine or a protease cathepsin B.

In another exemplary embodiment of the invention, the amino acid sequence represented by EGLFG, which is the target sequence of glutamic acid (Glu, E) or the protease cathepsin B, is a part of the linker peptide that binds to a fatty acid, and in the CP2c-targeting peptide-amino acid conjugate according to the present invention, the amino acid sequence may be linked to lysine of the linker peptide represented by GₙKGₘ by a peptide bond between a functional group (NH₂) of lysine and an alpha or gamma carboxyl group of glutamic acid, and may be linked to a fatty acid by a peptide bond between a carboxyl group of the fatty acid and an amino group of the peptide.

During the synthesis of the CP2c-targeting peptide-fatty acid conjugate according to the present invention, the amino acid sequence represented by EGLFG, which is the target sequence of glutamic acid (Glu, E) or the protease cathepsin B, first binds to the linker peptide represented by GnKGm, and then may be linked to a fatty acid, but is not limited to the synthesis order.

Meanwhile, for the CP2c-targeting peptide-fatty acid conjugate used in the present invention, the N- and/or C-terminus of the peptide may be modified in order to obtain the improved stability, enhanced pharmacological properties (half-life, absorbability, potency, efficacy, and the like), altered specificity (for example, broad biological activity spectrum), and reduced antigenicity of the peptide. Preferably, the above modification may be in a form in which an acetyl group, a fluorenyl methoxy carbonyl group, an amide group, a formyl group, a myristyl group, a stearyl group, or polyethylene glycol (PEG) binds to the N- and/or C-terminus of the peptide, but the modification of the peptide may particularly include any component that can improve the stability of the peptide without limitation. As used herein, the term "stability" refers not only to *in vivo* stability that protects the peptides of the invention from attack of a proteolytic enzyme *in vivo,* but also to storage stability (for example, room-temperature storage stability). Since the N-terminus of the peptide binds to an unsaturated fatty acid in the CP2c-targeting peptide-fatty acid conjugate according to the present invention, the CP2c-targeting peptide-fatty acid conjugate may have a structure in which only the C-terminus is modified with an amide group.

The structure of the CP2c-targeting peptide according to exemplary embodiments of the present invention is as follows:

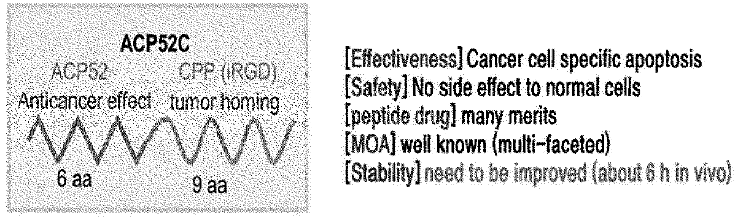
ACP52C
ACP52 CPP (iRGD)
Anticancer effect tumor homing
6 aa 9 aa
[Effectiveness] Cancer cell specific apoptosis
[Safety] No side effect to normal cells
[peptide drug] many merits
[MOA] well known (multi-faceted)
[Stability] need to be improved (about 6 h in vivo)

For example, in the CP2c-targeting peptide-fatty acid conjugate according to the present invention, the structures of ACP52 and ACP52C are specifically as follows: 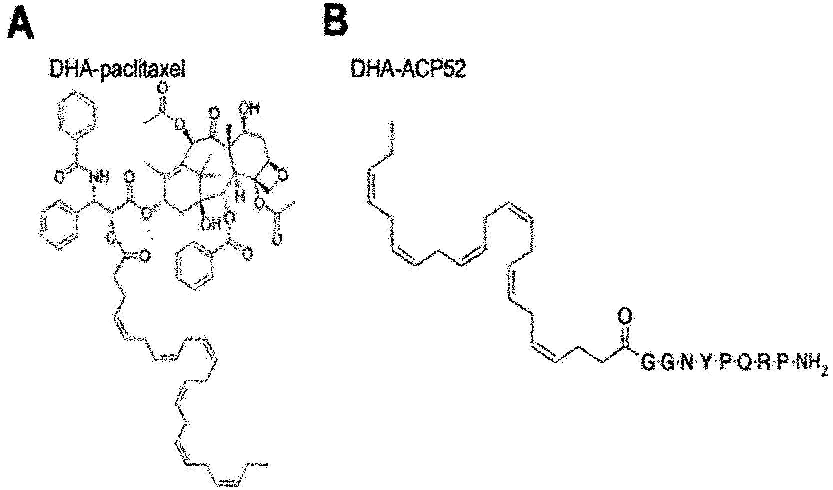 Bradley et al., (2001)

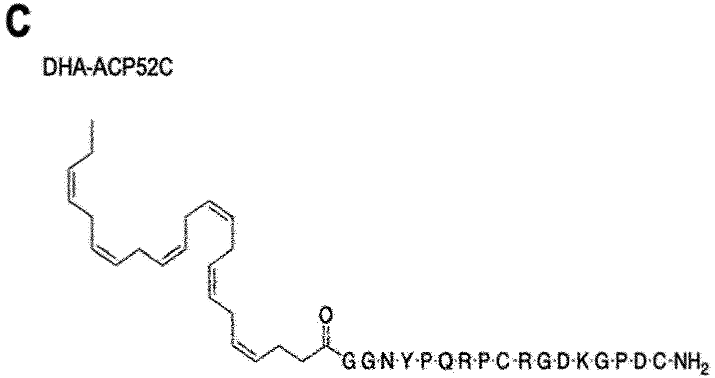

In the exemplary embodiments of the present invention, four synthesized CP2c-targeting peptide-fatty acid conjugates were used *in vivo* to analyze in vivo stability, anticancer efficacy, tumor metastasis inhibitory efficacy and physiological toxicity in liver cancer cell xenograft (Hep3B) and breast cancer cell xenograft (MDA-MB-231) mouse models, and as a result, it was confirmed that *in vivo* stability and tumor-inhibitory and tumor metastasis-inhibitory effects were excellent, and it was confirmed that the CP2c-targeting peptide-fatty acid conjugate is a safe substance that was not toxic to the organism.

Accordingly, the present invention also provides a pharmaceutical composition and a health functional food composition for preventing, ameliorating, or treating cancer, including the CP2c-targeting peptide-fatty acid conjugate according to the present invention as an active ingredient.

In the present invention, the cancer may also include drug-resistant cancer exhibiting resistance to anticancer agents, particularly, anticancer agents based on the CP2c-targeting peptide-fatty acid conjugate according to the present invention.

According to an exemplary embodiment of the present invention, the cancer includes gastric cancer, lung cancer, non-small cell lung cancer, breast cancer, ovarian cancer, liver cancer, bronchial cancer, nasopharyngeal cancer, laryngeal cancer, pancreatic cancer, bladder cancer, colorectal cancer, colon cancer, cervical cancer, bone cancer, non-small cell bone cancer, hematologic malignancies, skin cancer (melanoma, and the like), head or neck cancer, ueterine cancer, rectal cancer, perianal cancer, colon cancer, fallopian tube cancer, endometrial cancer, vaginal cancer, vulva cancer, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or hydroureter cancer, renal cell carcinoma, renal pelvic carcinoma, polyploid carcinoma, salivary gland cancer, sarcoma cancer, pseudomyxoma, hepatoblastoma, testicular cancer, glioblastoma, cheilocarcinoma, ovarian germ cell tumors, basal cell carcinoma, multiple myeloma, gallbladder cancer, choroidal melanoma, cancer of the ampulla of Vater, peritoneal cancer, adrenal cancer, tongue cancer, small cell cancer, pediatric lymphoma, neuroblastoma, duodenal cancer, ureteral cancer, astrocytoma, meningioma, renal pelvis cancer, pudendum cancer, thymus cancer, central nervous system (CNS) tumors, primary central nervous system lymphoma, spinal cord tumors, brain stem neuroglioma, and pituitary adenoma, but is not limited thereto.

As used herein, the term "treatment" refers to all actions that ameliorate or beneficially change symptoms of cancer by administering the peptide according to the present invention or a pharmaceutical composition including the same.

In the present invention, the "containing as an active ingredient" refers to an amount sufficient to treat a disease at a reasonable benefit/risk ratio applicable to medical treatment, and the level of the effective dosage can be determined according to the type and severity of disease of a patient, the activity of the drug, the drug sensitivity in a patient, the administration time, the administration pathway and release rate, the treatment duration, elements including drugs that are simultaneously used with the composition of the present invention, or other factors well-known in the medical field. The peptide according to the present invention or a pharmaceutical composition including the same may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by a person skilled in the art. The dosage and frequency of the pharmaceutical composition of the present invention are determined by the type of drug, which is an active ingredient, as well as various related factors such as the disease to be treated, the administration route, the age, sex, body weight of a patient, and the severity of the disease.

Therefore, the pharmaceutical composition according to the present invention may include various pharmaceutically acceptable carriers as long as the peptide according to the present invention is contained as an active ingredient. As the pharmaceutically acceptable carrier, a binder, a lubricant, a disintegrant, an excipient, a solubilizing agent, a dispersing agent, a stabilizer, a suspending agent, a colorant, a flavoring agent, and the like may be used when used for oral administration, in the case of injection, a buffering agent, a preservative, an analgesic, a solubilizer, an isotonic agent, a stabilizer, and the like may be mixed and used, and in the case of topical administration, a base, an excipient, lubricant, a preservative, and the like may be used. The formulation of the pharmaceutical composition of the present invention may be variously prepared by mixing the pharmaceutical composition of the present invention with a pharmaceutically acceptable carrier as described above. For example, the formulation may be prepared in the form of a tablet, a troche, a capsule, an elixir, a suspension, a syrup, a wafer, and the like when orally administered, and in the case of injection, the injection may be formulated into unit dosage ampoules or in multiple dosage forms. The pharmaceutical composition of the present invention may be formulated into other solutions, suspensions, tablets, pills, capsules, sustained-release preparations, and the like.

Meanwhile, as an example of suitable carriers, excipients, and diluents for formulation, it is possible to use lactose, dextrose, sucrose, sorbitol, mannitol, xylitol, erythritol, maltitol, starch, acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, or the like. Further, the pharmaceutical composition of the present invention may additionally include a filler, an anticoagulant, a lubricant, a wetting agent, a flavoring agent, an antiseptic, and the like.

The pharmaceutical composition of the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including type of disease of patients, the severity of disease, the activity of drugs, sensitivity to drugs, administration time, administration route, excretion rate, treatment period, and simultaneously used drugs, and other factors well known in the medical field. The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by those skilled in the art.

As used herein, the term "administration" refers to the introduction of a predetermined substance, that is, the peptide derivative according to the present invention or a pharmaceutical composition containing the same, into a subject by any suitable method, and the route of administration may be through any common route as long as a drug can reach a target tissue. For example, the route of administration may be intraperitoneal administration, intravenous administration, intramuscular administration, subcutaneous administration, intradermal administration, oral administration, topical administration, intranasal administration, intrapulmonary administration, rectal administration, and the like, but is not limited thereto. However, since the peptide is digested during oral administration, it is preferable for the oral composition to be formulated with an active agent coated or otherwise protected from gastric degradation. Preferably, the pharmaceutical composition of the present invention may be administered in the form of an injection. In addition, the pharmaceutical composition according to the present invention may also be administered by any device which may allow an active material to move to a target cell.

Since the peptide according to the present invention can also be contained as an active ingredient in a health functional food composition, a food composition according to the present invention may be prepared in the form of a composition by being mixed with a known active ingredient known to have an anticancer function and may additionally include a sitologically acceptable food supplemental additive. The health functional food composition of the present invention includes compositions for all types of food such as a functional food, a nutritional supplement, a health food, and a food additive.

These types of food composition may be prepared in various forms by typical methods publicly known in the art. For example, a health food may be prepared in various forms such as tablets, pills, powders, capsules, gums, vitamin complexes, juices and drinks, and the food composition including the peptide according to the present invention as an active ingredient may be ingested in the form of granules, capsules, or powders. The food composition of the present invention may include an ingredient typically added during the preparation of food, and include, for example, proteins, carbohydrates, fats, nutrients, and seasonings. For example, when the food composition of the present invention is prepared as a drink, the composition may additionally include citric acid, high fructose corn syrup, sugar, sucrose, acetic acid, malic acid, a fruit juice, a jujube extract, a licorice extract, and the like in addition to the peptide of the present invention. Furthermore, the food composition of the present invention may include, as food supplemental additive, a typical food additive in the art, for example, a flavoring agent, a savoring agent, a colorant, a filler, a stabilizer, and the like. Further, the food composition of the present invention may contain various flavoring agents or natural carbohydrates, and the like as an additional ingredient, as in a typical beverage. Specific examples of natural carbohydrates include, for example, monosaccharides such as glucose and fructose, disaccharides such as maltose and sucrose, typical sugars such as polysaccharides such as dextrin and cyclodextrin, and sugar alcohols such as xylitol, sorbitol and erythritol. As flavoring agents in addition to those described above, a natural flavoring agent [thaumatin, a stevia extract (for example, rebaudioside A, glycyrrhizin, and the like)], and a synthetic flavoring agent (saccharin, aspartame, and the like) may be included.

Furthermore, the food composition of the present invention may contain various nutrients, vitamins, minerals (electrolytes), savoring agents such as synthetic savoring agents and natural savoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like. These ingredients may be used either alone or in combinations thereof.

Hereinafter, the present invention will be described in more detail through Examples, and the following Examples are provided to help better understand the present invention and are not intended to limit the scope of the present invention.

### [Advantageous Effects]

In the present invention, in order to identify a final candidate substance that improved *in vivo* stability of a transcription factor CP2c-targeting peptide ACP52C, which has shown an effect as a pan anticancer agent, a fatty acid was linked to the CP2c-targeting peptide lead substance ACP52C to confirm the *in vivo* stability and anticancer efficacy in a cancer cell xenograft mouse model. Further, the cancer cell-specific anticancer efficacy of a final anticancer candidate substance was confirmed in various cancer cells and normal cells.

### [Description of Drawings]

FIG. 1 illustrates the experimental results showing the process of identifying a peptide (PEP #5-2) that interferes with the CP2c complex formation and its DNA binding capability.
FIG. 2 is a graph showing the GI₅₀ values in various cancer cell lines by the treatment of ACP52C.
FIG. 3 illustrates colony formation ability of MDA-MB-231 and HepG2 cell lines by the treatment of ACP52C and/or CP2c overexpression.
FIG. 4 is a set of FACS views showing cell cycle arrest at G2/M stage, cell death induction (subG1 cells), and the expression of p53 and subregulators which regulate cell cycle and apoptosis by ACP52C treatment.
FIG. 5 is a set of views illustrating induction of apoptosis by ACP52C treatment.
FIG. 6 illustrates the safety and stability analysis results of ACP52C.
FIG. 7 illustrates the structure and characteristics of ACP52C.
FIG. 8 illustrates the structures of a conjugate (ACP52CG) in which an albumin-affinity saturated fatty acid (palmitoyl acid) is bound to ACP52C and a conjugate (ACP52CK) in which an albumin-affinity saturated fatty acid (palmitoyl acid) and a cathepsin B sensitive peptide are bound to ACP52C in order to improve *in vivo* stability.
FIG. 9 illustrates the cell growth analysis results by the treatment with C16 saturated fatty acid binding peptide (ACP52C; ACP52CG; ACP52CK).
FIG. 10 illustrates the cell growth analysis results in a normal cell line (hMSC), a non-tumorigenic transformed cell line (BEAS-2B), and cancer cell lines (Hep3B, MDA-MB-231, and HepG2) by the treatment with a C16 saturated fatty acid binding peptide (ACP52C; ACP52CG; ACP52CK).
FIG. 11 illustrates the anticancer effect analysis results of ACP52CG and ACP52CK in a Hep3B cell line xenograft mouse model.
FIG. 12 illustrates the cell permeability and intracellular function of a natural unsaturated fatty acid, omega-3, which is harmless to the human body.
FIG. 13 illustrates the cell growth analysis results in a normal cell line (hMSC), a non-tumorigenic transformed cell line (BEAS-2B), and cancer cell lines (Hep3B, MDA-MB-231, and HepG2) by the treatment with an unsaturated fatty acid DHA (0 to 100 µM).
FIG. 14 illustrates the cell growth analysis results in cancer cell lines (Hep3B and MDA-MB-231) by the treatment with 10 µM or less of an unsaturated fatty acid DHA.
FIG. 15 illustrates the cell growth analysis results in a normal cell line (hMSC), a non-tumorigenic transformed cell line (BEAS-2B), and cancer cell lines (Hep3B, MDA-MB-231, and HepG2) by the combined treatment with an unsaturated fatty acid DHA and ACP52C.
FIG. 16 illustrates the structures of an exemplary drug (DHA-paclitaxel) to which an unsaturated fatty acid DHA has been conjugated and ACP52-derived peptides DHA-ACP52C and DHA-ACP52C to which the unsaturated fatty acid DHA has been conjugated, as an effort to improve *in vivo* stability.
FIG. 17 illustrates the cell growth analysis results in a normal cell line (hMSC), a non-tumorigenic transformed cell line (BEAS-2B), and cancer cell lines (MDA-MB-231 and HepG2) by the treatment with a DHA-ACP52 or DHA-ACP52C peptide.
FIG. 18 illustrates the protein expression levels of CP2c, MDM2, YY1, p53, p63, and p73 in cancer cells (MDA-MB-231 and HepG2) by the treatment with ACP52C or DHA-ACP52C.
FIG. 19 illustrates the changes in cell cycle and the protein expression levels of apoptosis regulators in cancer cells (MDA-MB-231 and HepG2) by the treatment with ACP52C or DHA-ACP52C.
FIG. 20 illustrates the anticancer effects in a Hep3B xenograft mouse model, where sorafenib, DHA-ACP52, or DHA-ACP52C was injected with 3-day intervals via tail vein.
FIG. 21 illustrates the body weight, and hematological and histological data in a Hep3B xenograft mouse model, where sorafenib, DHA-ACP52, or DHA-ACP52C was injected with 3-day intervals via tail vein.
FIG. 22 illustrates the anticancer effect of ACP52C, DHA-ACP52, and DHA-ACP52C in a Hep3B xenograft mouse model, where sorafenib, DHA-ACP52, or DHA-ACP52C was injected with 5-day intervals via tail vein.
FIG. 23 illustrates the body weight, and hematological and histological data in a Hep3B xenograft mouse model, where sorafenib, DHA-ACP52, or DHA-ACP52C was injected with 5-day intervals via tail vein.

### [Modes of the Invention]

Hereinafter, the present invention will be described in more detail through Examples, and the following Examples are provided to help better understand the present invention and are not intended to limit the scope of the present invention.

### [Preparation Example 1] Preparation of CP2c-targeting peptide to which cell-penetrating peptide is bound

A transcription factor CP2c is known to be overexpressed in various cancers, and a study conducted by a US research team has reported that suppression of CP2c expression in liver cancer cell lines suppresses cell growth, and when CP2c is overexpressed, the malignancy and metastasis of cancer occur (Grant et al., Antiproliferative small-molecule inhibitors of transcription factor LSF reveal oncogene addiction to LSF in hepatocellular carcinoma. Proc Natl Acad Sci USA 2012, 109: 4503-4508).

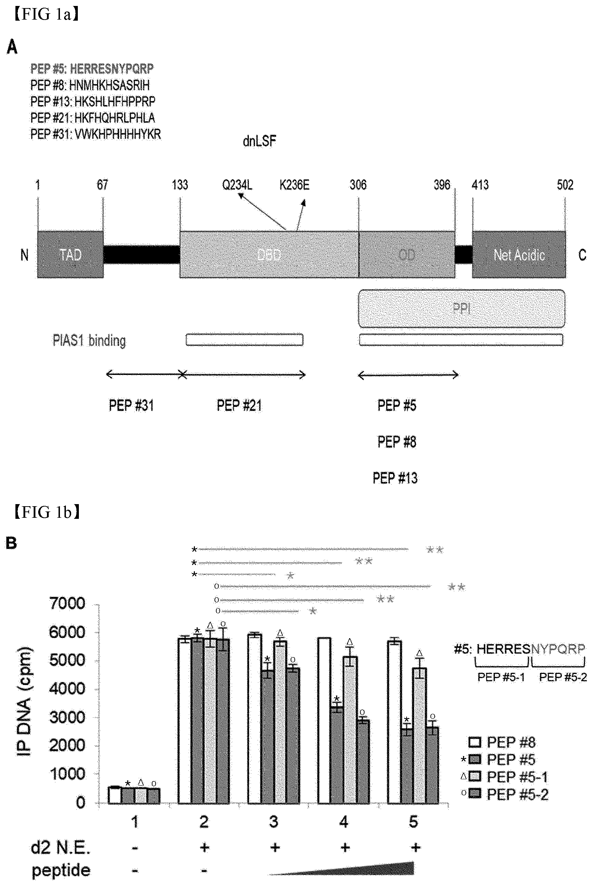

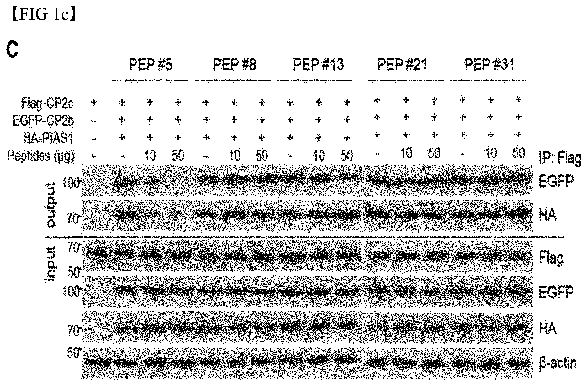

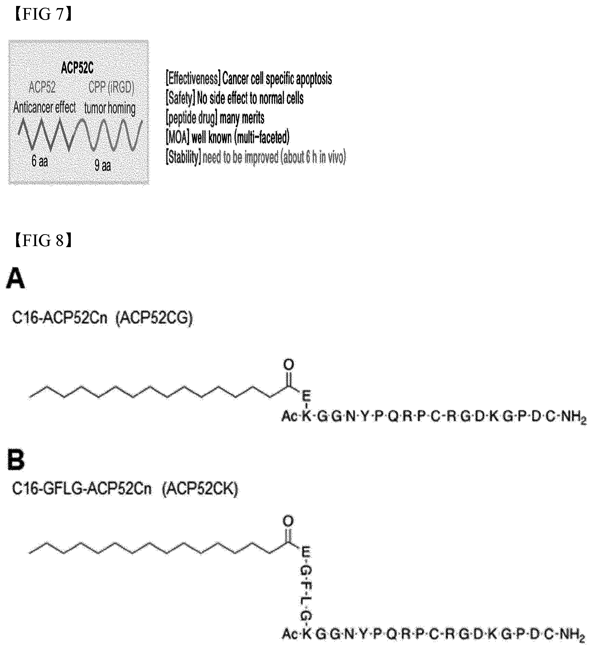

The present inventors identified peptides that bind to the transcription factor CP2c (also known as Tfcp2, LSF, LBP1, UBP1, and the like) by the phage display method (Kang et al., Identification and characterization of four novel peptide motifs that recognize distinct regions of the transcription factor CP2. FEBS 2005, 272:1265-1277), and it was confirmed that one peptide (PEP #5, SEQ ID NO: 1) among them interferes with DNA binding of the CP2c transcription factor complexes [CP2c homotetramer and the CBP (CP2c, CP2b, and PIAS1) heterohexamer] by suppressing the CP2c transcription factor complex formation. In particular, it was confirmed that PEP #5-2 (SEQ ID NO: 2) consisting of 6 amino acids at the carboxyl terminus of PEP #5 also showed the same effect as PEP #5, and this was selected as a CP2c-targeting peptide (FIG. 1). Since PEP #5-2 cannot enter cells by itself, in order to improve cell permeability of Pep #5-2, a cell-penetrating peptide (CPP), an internalizing RGD (iRGD) which is a peptide consisting of 9 amino acids (9aa) 'Cys-Arg-Gly-Asp-Lys-Gly-Pro-Asp-Cys (CRGDKGPDC, SEQ ID NO: 3)', was bound to the CP2c-targeting peptide and named it as ACP52C (FIG. 7).

### [Example 1] Confirmation of anticancer effect of ACP52C

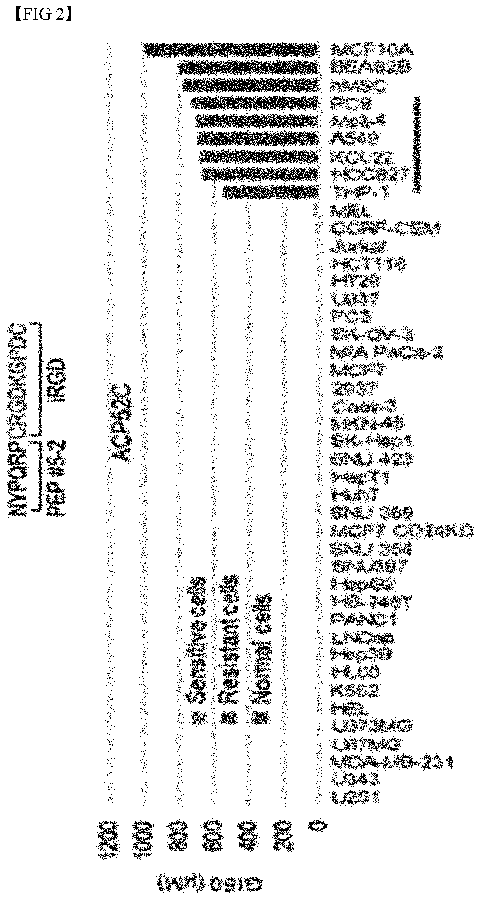

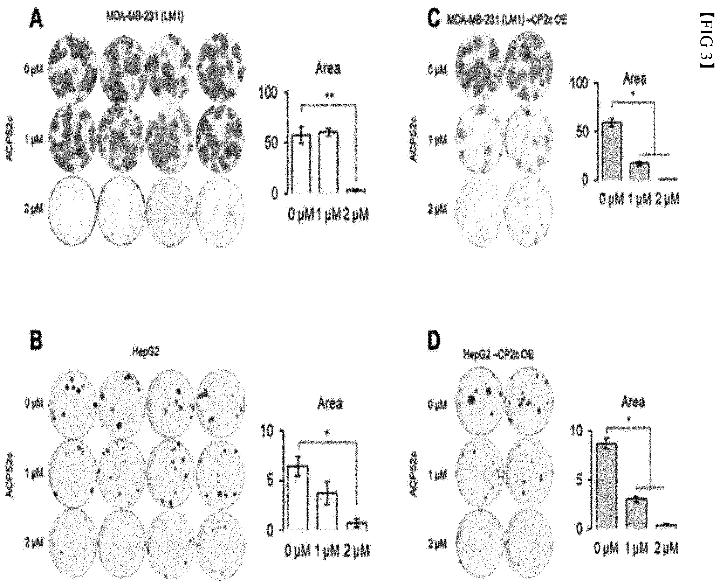

The selected peptide indirectly interferes with the DNA binding of CP2c by binding to CP2c to suppress the formation of the CP2c transcription factor complexes (CP2c homotetramer and CP2c/CP2b/PIAS1 heterohexamer). By analyzing the anticancer efficacy of ACP52C in various cancer cell lines, ACP52C was confirmed to show cancer cell-specific growth suppression and cell death efficacies (FIG. 2). ACP52C also efficiently suppressed the colony formation ability in MDA-MB-231 and HepG2 cell lines. Moreover, when CP2c is overexpressed, the colony formation ability is suppressed more efficiently, confirming that the efficacy of ACP52C is dependent on the expression level of CP2c (FIG. 3). This result is consistent with a phenomenon in which the expression of CP2c in most cancer cell lines is 50-fold or higher than in normal cells and ACP52C shows oncogenic addiction in cancer cells.

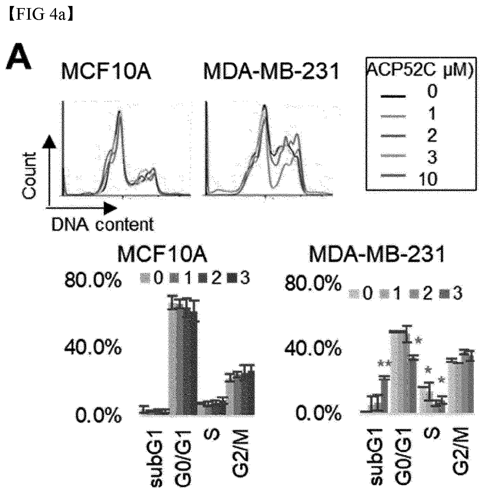

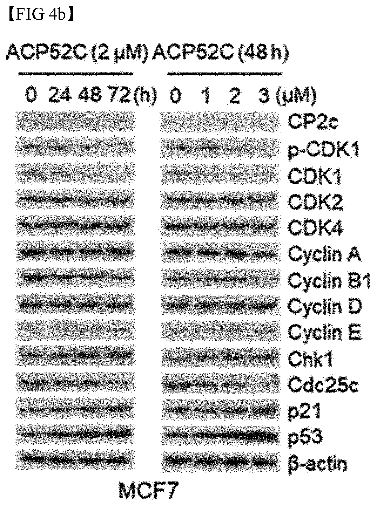

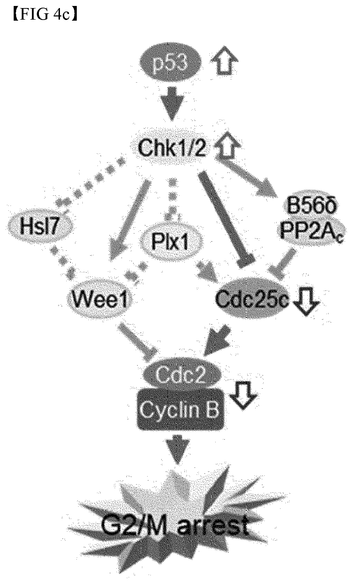

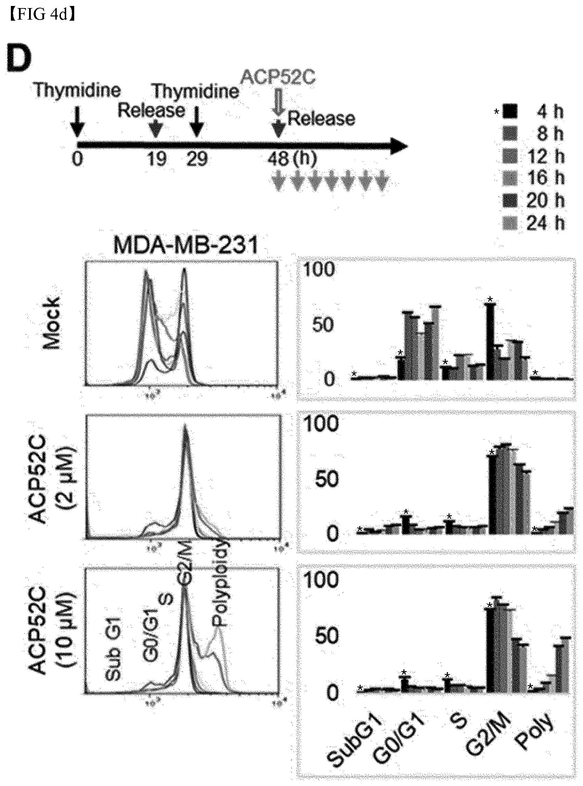

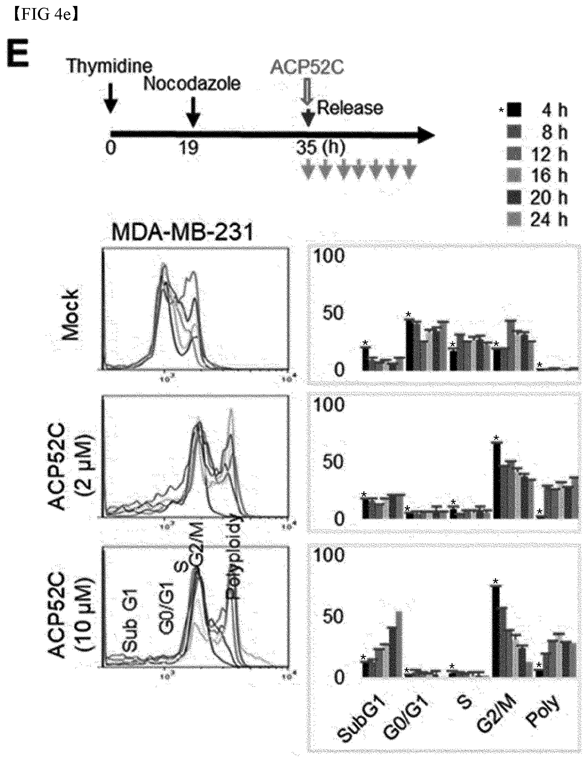

By analyzing the ACP52C treatment-dependent cell cycle changes by FACS, it was confirmed that ACP52C induced cell cycle arrest, increasing cells at G2/M and subG1 phases, in the MDA-MB-231 cell line whereas ACP52C exerted no effect on cell cycle progression in the MCF10A, a non-tumorigenetic breast epithelial cell line. In addition, it was verified that expression of Cdc2 (CDK1) and Cyclin B1 was decreased via the p53/CHK1/CDC25c axis in the ACP52C-treated MCF7 cells, where the G2/M phase arrest occurred. To analyze more accurately about the ACP52C effect on cell cycle progression, when ACP52C was treated to the cells released from the double thymidine block method-mediated G1/S phase synchronization, it was confirmed that ACP52C induced a G2/M phase arrest accompanying polyploid chromosome formation. Meanwhile, when ACP52C was treated to the cells released from the nocodazole treatment-mediated G2/M phase synchronization, it was confirmed that the number of subG1 cells was increased over time in addition to the G2/M phase arrest and polyploid formation (FIG. 4). Therefore, it could be presumed that cell death induction by ACP52C treatment occurs sequentially through G2/M cell cycle arrest, polyploid formation, and subG1 cell formation.

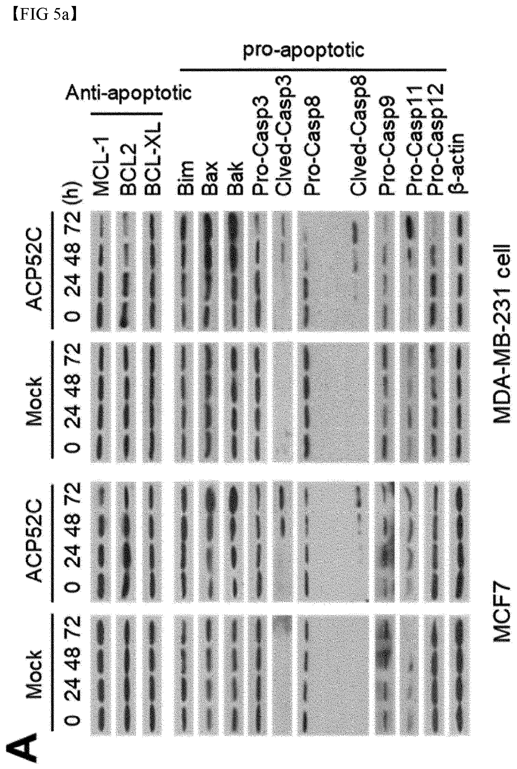

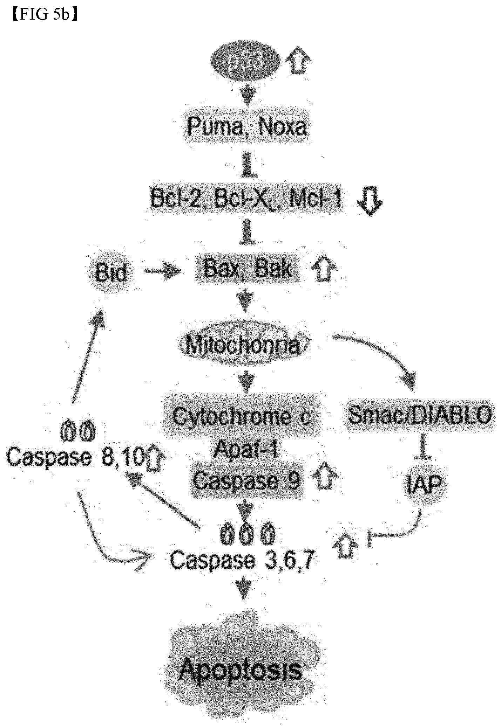

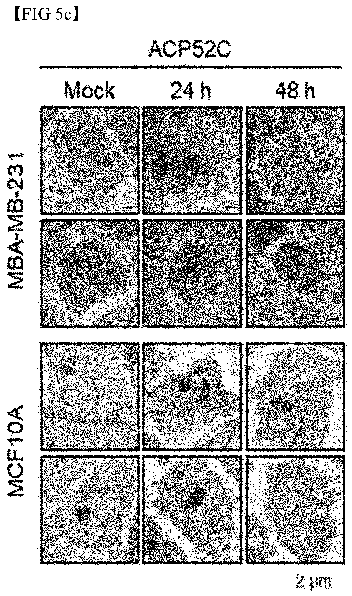

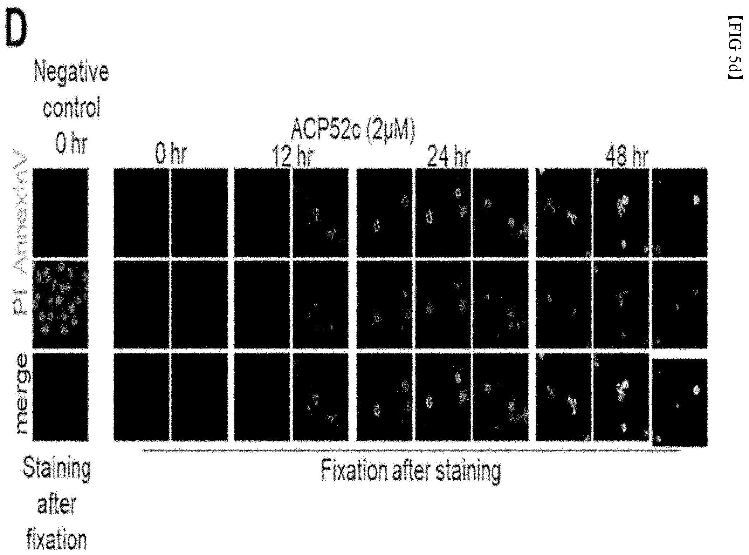

By analyzing the expression levels of apoptosis-related proteins in mock (non-treated) and ACP52-treated cells proteins over time by western blotting, it was confirmed that ACP52C treatment induced cell death via apoptosis, increasing the expression levels of a pro-apoptotic marker proteins, decreasing the expression levels of a anti-apoptotic marker proteins, and activating the caspase cascade (FIGS. 5A and 5B). When analyzed the subcellular characteristics of cells treated with to ACP52C by an electron microscopy, the prominent characteristics of apoptosis, like the condensation of cell nuclei, was observed in MDA-MB-231 cells, but not at all in MCF10A cells (FIG. 5C). The fact that ACP52C induces cell death through apoptosis was also confirmed by the Annexin V/PI staining method (FIG. 5D). Therefore, it could be presumed that ACP52C shows cell death efficacy through the apoptosis process.

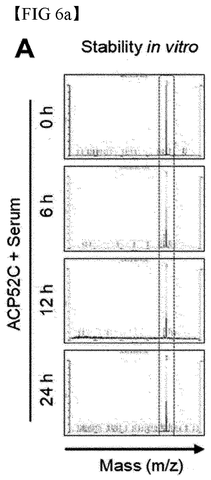

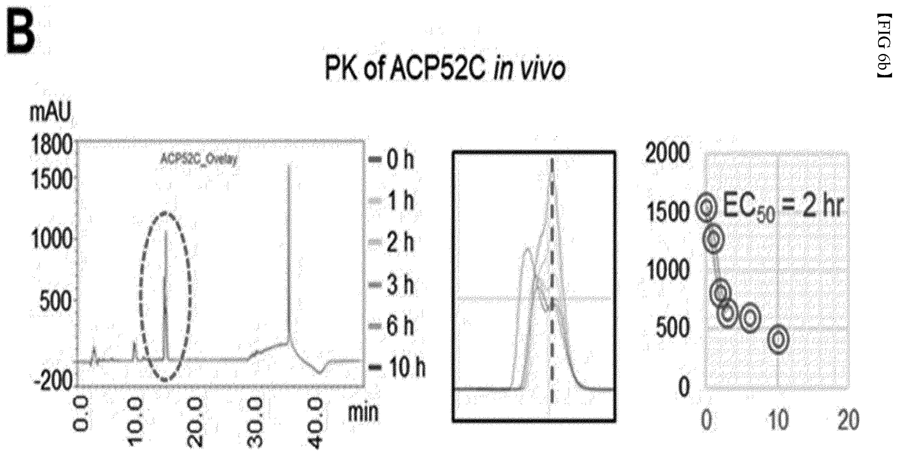

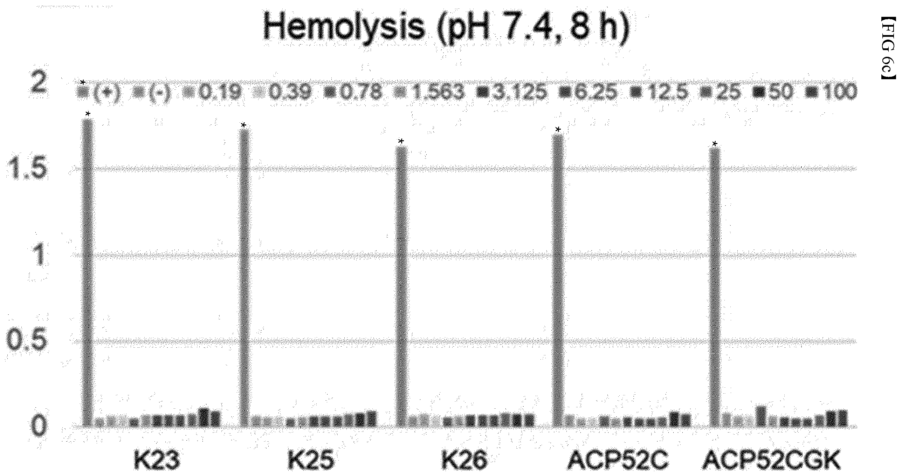

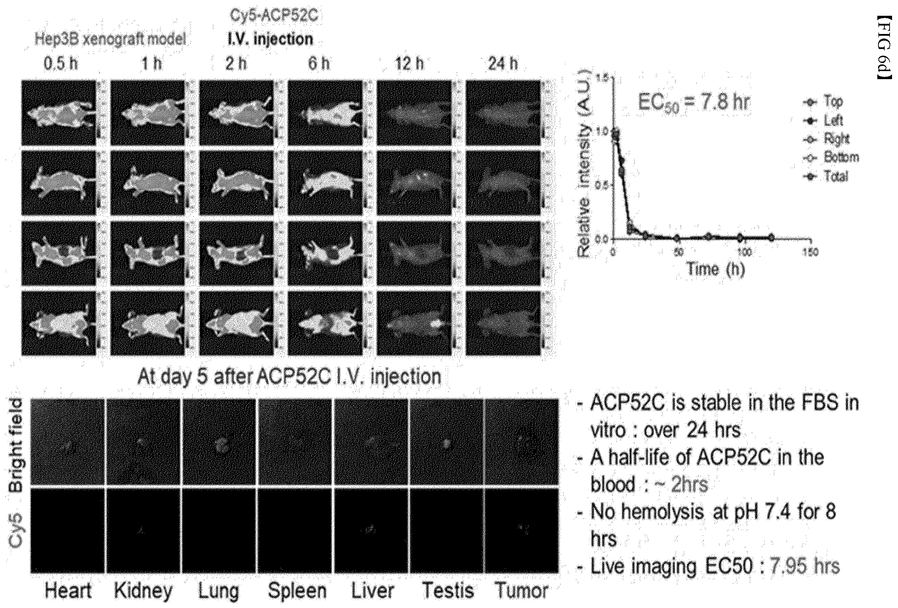

The following methods were used to confirm the stability and safety of the cancer cell-specific cell death-inducing ACP52C. At first, when the half-life of ACP52C in serum (*in vitro*) and blood (*in vivo*) was measured by HPLC, ACP52C was stable *in vitro* for up to 24 hours whereas it was degraded to an half in 2 hours *in vivo* (FIGS. 6A and 6B). Hemolysis was not appeared when human erythrocytes were treated with ACP52C at pH 7.4 for 8 hours (FIG. 6C). Meanwhile, when a Cy5-labeled peptide (Cy5-ACP52C) was injected into a Hep3B cell line xenograft mouse via tail vein and then the live imaging was performed over time, most of the injected ACP52 disappeared within 24 hours although the peptide could observable in the kidneys, liver, and tumor after 5 days. The half-life of ACP52C in vivo is measured to be about 7.95 hours (FIG. 6D). It is presumed that ACP52C is subjected to renal secretion rather than degradation by proteases, since ACP52C (a peptide consisted of 15 amino acids) is short enough to be easily secreted from the kidneys.

### [Preparation Example 2] Synthesis of CP2c-targeting peptide-saturated fatty acid conjugate

The results of Example 1 Indicate that ACP52C caused cancer cell-specific cell death through apoptosis with rarely affecting normal cells. However, since *in vivo* stability needs to be secured in order to become a candidate substance for an anticancer agent, it was intended to use an albumin-affinity fatty acid-conjugated peptide as a method for achieving this. Albumin is a stable protein that is abundant in blood and binds well to fatty acids and thus, when a drug is bound to fatty acids, it is proposed that the renal secretion of the drug could be inhibited by binding to albumin (Sleep et al., Albumin as a versatile platform for drug half-life extension. Biochim Biophys Acta 2013, 1830: 5526-5534; van Witteloostuijin et al., Half-life extension of biopharmaceuticals using chemical methods: alternatives to PEGylation. ChemMedChem 2016, 11: 2474-2495). In addition, lilaglutide, which is clinically used as a therapeutic agent for diabetes, has also had C16 palmitoyl acid bound to a peptide in order to secure stability in blood. Based on these facts, a peptide C16-ACP52C (ACP52CG) in which C16 palmitoyl acid was bound to the N-terminus of ACP52C was synthesized (FIG. 8A). On the other hand, we speculated that the conjugated fatty acids to the ACP52c peptides may inhibit the nuclear transfer or CP2c targeting of the peptides and thus they should be removed within the cells after transfer into the cancer cell. Accordingly, C16-GFLG-ACP52Cn (ACP52CK), a peptide in which a cathepsin B targeting sequence 'GFLG' was inserted between the fatty acid and ACP52C, was also synthesized (FIG. 8B). Since cathepsin B peptidase activity is known to be high around cancer tissue, ACP52CK is expected to be cleaved at 'GFLG' by cathepsin B around the cancer tissue, generating ACP52C with no fatty acids.

### [Example 2] Confirmation of in vitro anticancer effect of CP2c-targeting peptide-fatty acid conjugate

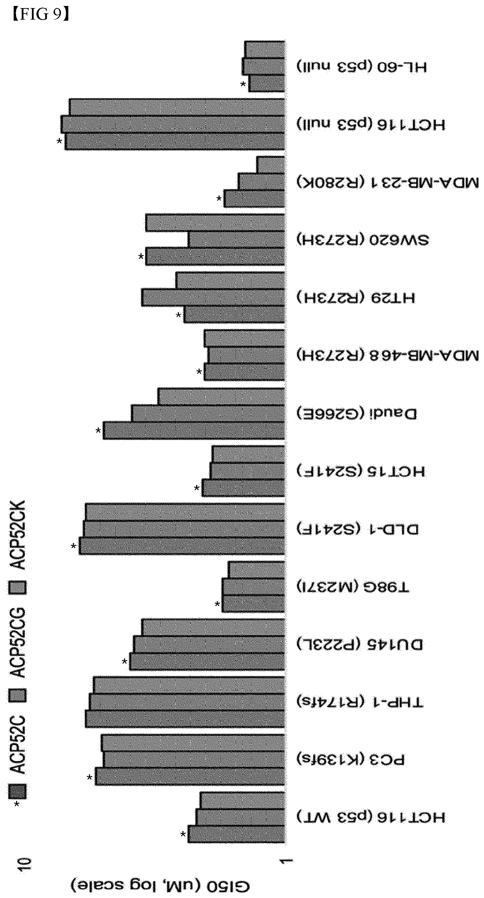

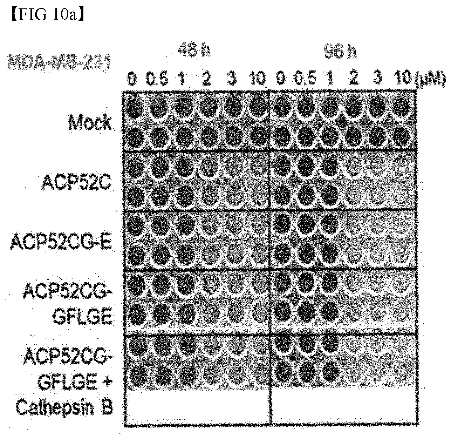

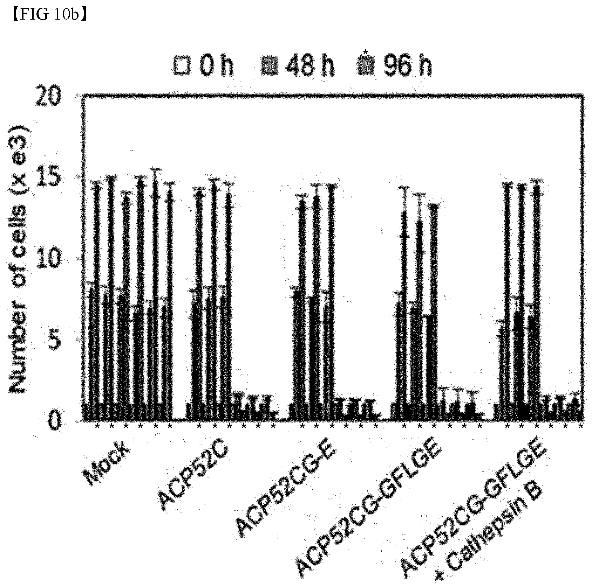

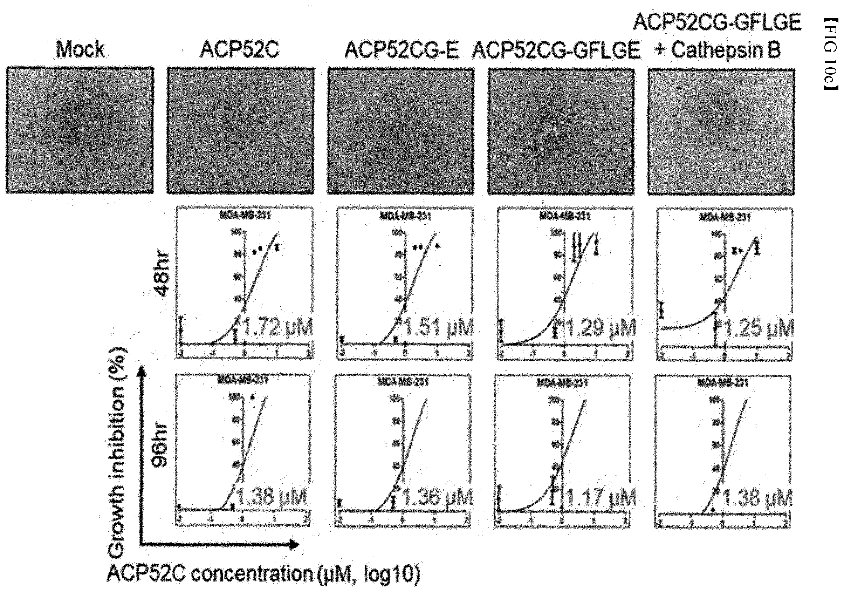

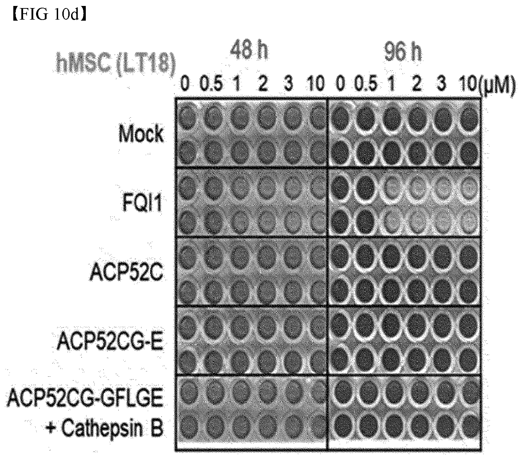

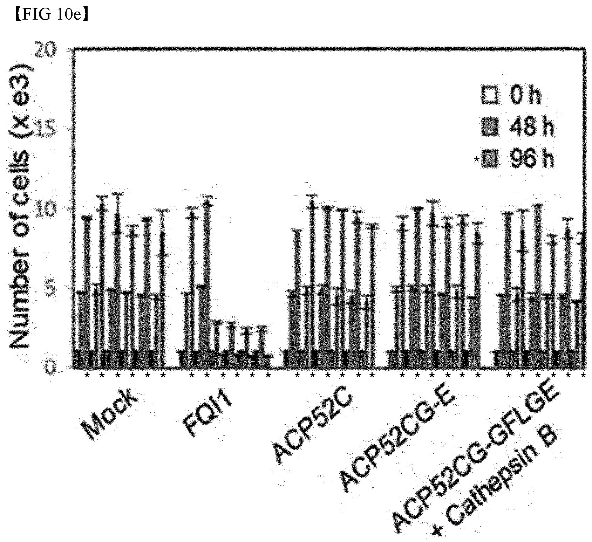

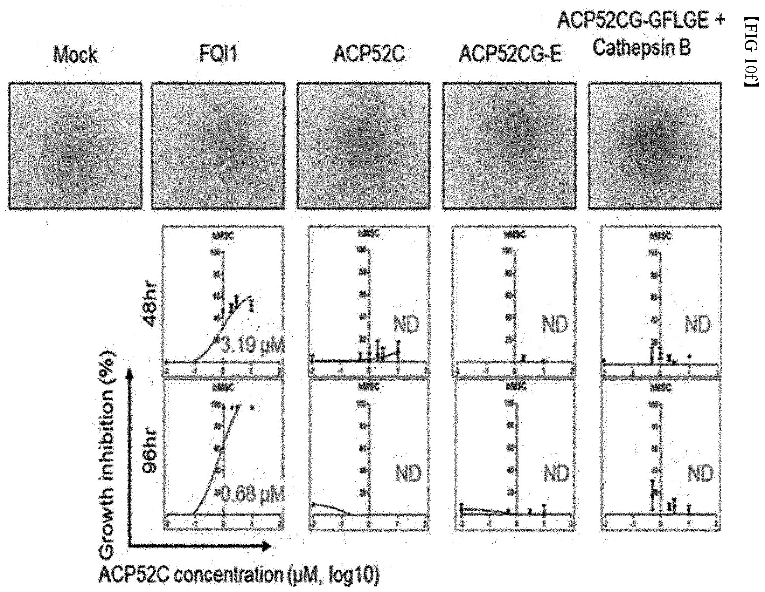

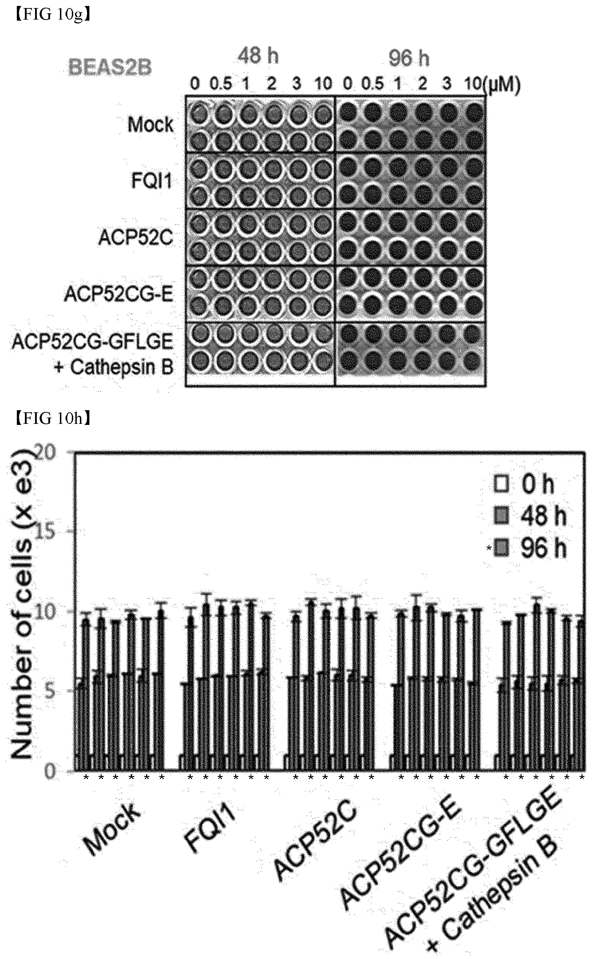

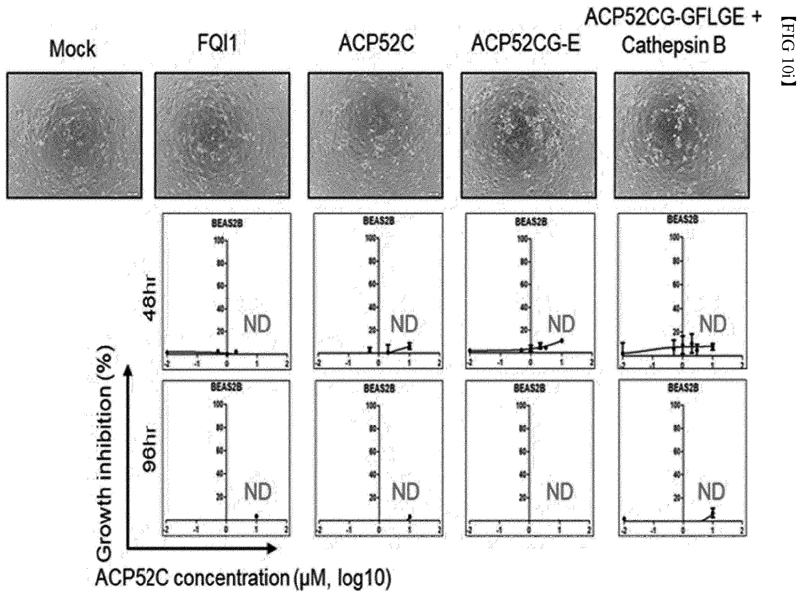

When both ACP52CG and ACP52CK were tested their cell grow inhibition effect in various cancer cell lines by MTT [(4,5-dimethylthiazol-2-yl)-2,5-diphenyltetrazolium bromide] assay, they showed a similar GI₅₀ values (50% growth inhibitory concentration) to that of ACP52C (FIG. 9). As an example thereof, FIG. 10 illustrates cell numbers at 48 hours and 96 hours after treatment of each drug at different concentrations in a breast cancer cell line (MDA-MB-231), normal human mesenchymal stem cells (hMSCs, LT18), and a non-tumorigenic lung cell line (BEAS-2B), cell growth inhibition graphs to get GI₅₀ values, and representative photographs of cells. It was confirmed that both ACP52CG and ACP52CK exhibited similar cell death induction efficacy in various cancer cells including MDA-MB-231 or BEAS-2B to a positive control, ACP52C, whereas ACP52CG and ACP52CK exerted a less cytotoxic effect in the hMSC cell line (LT18) than ACP52C. Meanwhile, since ACP52CK did not show any further cell death induction effect than ACP52CG, it was confirmed that cathepsin B targeting or fatty acids conjugated to the ACP52 peptide did not affect the anticancer activity of ACP52C.

### [Example 3] Tests of CP2c-targeting peptide-fatty acid conjugates on tumor growth inhibition and general physiological toxicity in various cancer cell line xenograft mouse models

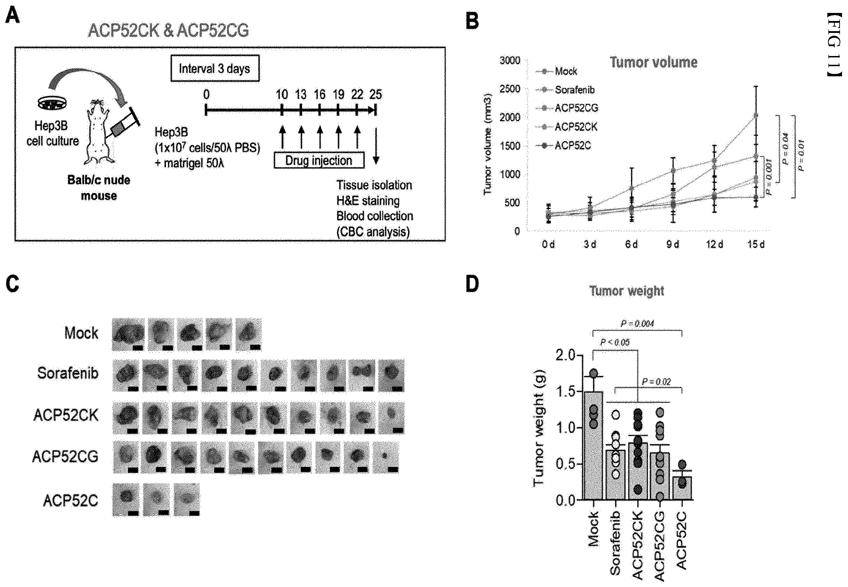

In order to confirm the anticancer efficacy of ACP52CG and ACP52CK *in vivo,* following groups of treatment were performed in the Hep3B xenograft mouse models by tail vein injection of a total of 5 times at 3-day intervals; a negative control (saline; mock), a positive control (sorafenib, 10 mg/kg and ACP52C, 2 mg/kg), and an experimental group (ACP52CK, 5 mg/kg and ACP52CG, 5 mg/kg). Although both ACP52CG and ACP52CK showed better anticancer efficacy in tumor size and weight when compared to a mock group (a negative control), the efficacy level was quite similar to or significantly less than that of sorafenib and ACP52C, respectively (FIG. 11).

The following hypothesis was established as a reason for which the anticancer effect of ACP52CG and ACP52CK is not as good as that of ACP52C. It was speculated that palmitic acids in the N-terminus of ACP52CG and ACP52CK could easily aggregates with each other to form a micelle structure, and thus palmitic acids within the micelle cannot bind to albumin, and ACP52CG and ACP52CK cannot penetrate into cell membrane (since the cell-penetrating peptide iRGD was located inside the micelle), suppressing cancer cell delivery of ACP52CG or ACP52CK.

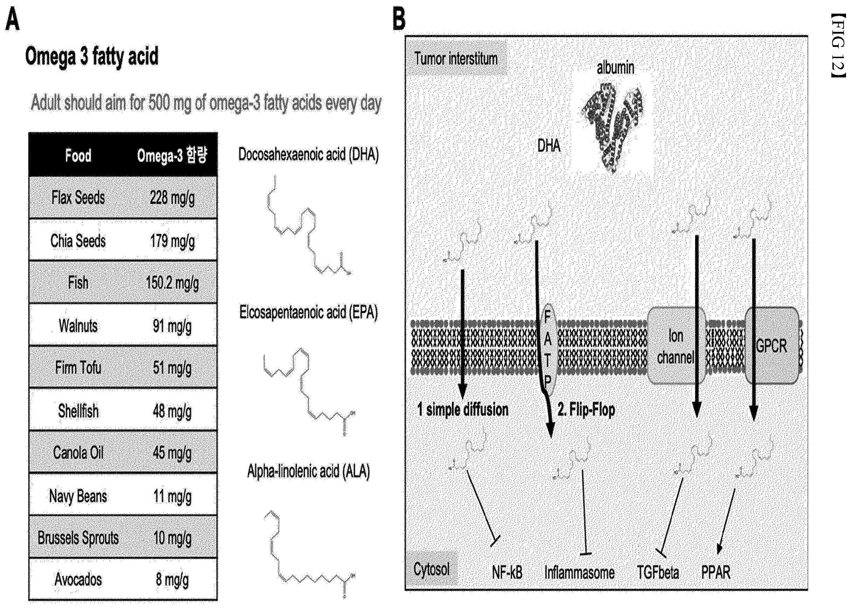

To overcome these problems, omega-3, which is an unsaturated fatty acid that cannot form a micelle, was employed. Omega 3, an unsaturated fatty acid with a double bond at the third carbon from the end of a carbon chain, is a substance that is easily found in nature, contained in a large amount in many foodstuffs, and one of the foods that are recommended for a healthy life, and is examples thereof include docosahexaenoic acid (DHA), eicosapentaenoic acid (EPA), alpha-linolenic acid (ALA), and the like (FIG. 12A). Unsaturated fatty acids have been shown to easily pass through cell membranes via various pathways and are known to regulate various signaling systems (Seitz and Ojima, Drug conjugates with polyunsaturated fatty acids. Drug Delivery in Oncology 2011, 3: 1-38; Endo et al., Cardioprotective mechanism of omega-3 polyunsaturated fatty acids. J Cardiol 2016, 67: 22-27) (FIG. 12B). In particular, it has been reported that DHA can be used as a biochemical precursor and energy source, and thus is harmless to the human body, easily binds to and passes through phospholipids of cell membranes, or regulates signaling systems from cell membrane structural proteins and fluids (Sauer et al., Uptake of plasma lipids by tissue-isolated hepatomas 7288CTC and 7777 in vivo. Br J Cancer 1992, 66: 290-296; Takahashi et al., Grammatikos et al., n-3 and n-6 fatty acid processing and growth effects in neoplastic and non-cancerous human mammary epithelial cell lines. Br J Cancer 1994, 70: 219-227). In particular, since it is known to have a potential therapeutic effect on cancer and neurodegenerative diseases (Youdim et al., Essential fatty acids and the brain: possible health implications. Int J Dev Neurosci 2000, 18: 383-399; Jaracz et al., Recent advances in tumor-targeting anticancer drug conjugates. Bioorg Med Chem 2005, 13: 5043-5054; Yu et al., Docohexaenoic acid suppresses neuroinflammatory responses and induces heme oxygenase-1 expression in BV-2 microglia: implications of antidepressant effects for omega-3 fatty acids. Neuropsychopharmacology 2010, 35: 2238-2248), DHA used alone in clinical trials. Accordingly, it was determined to explore the anticancer effect of a compound in which DHA binds to ACP52C.

### [Preparation Example 3] Synthesis of CP2c-targeting peptide-DHA conjugate

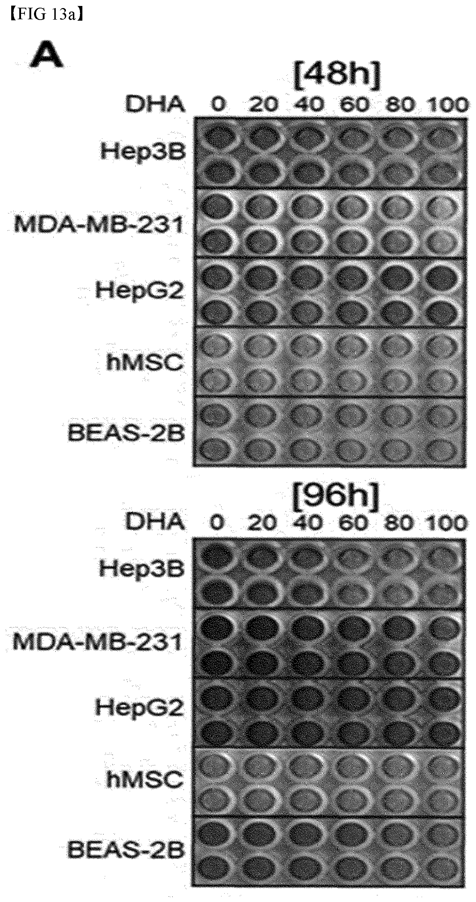

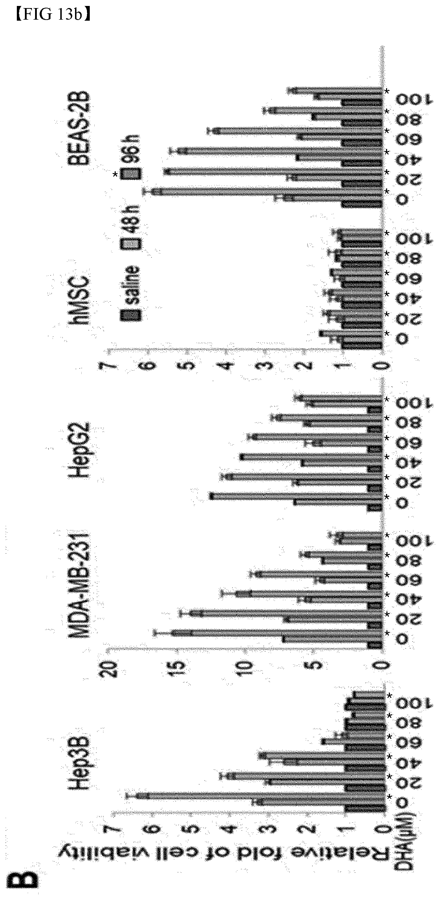

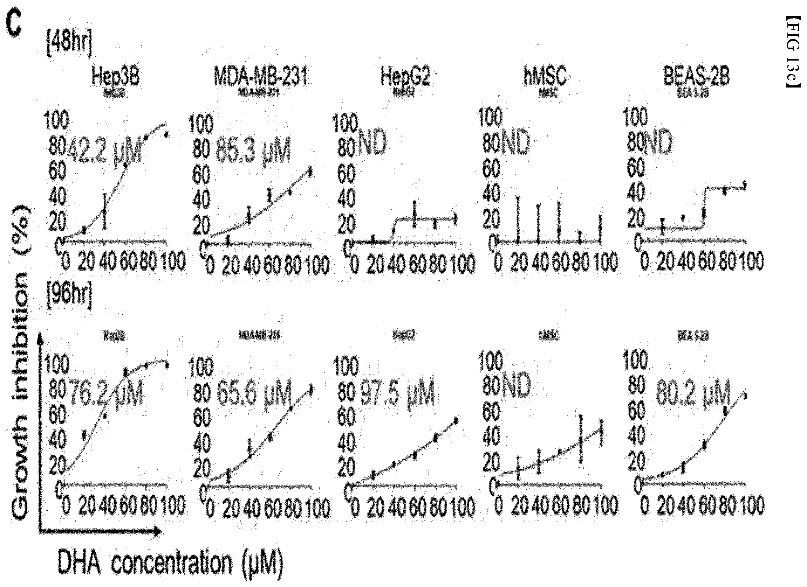

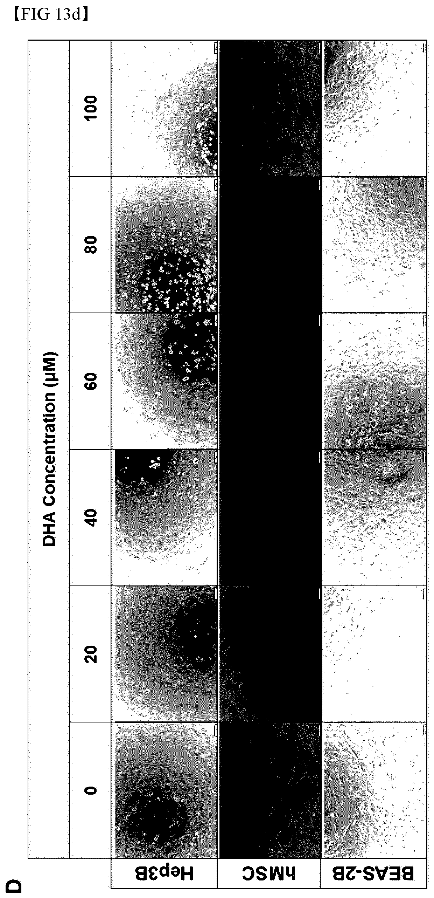

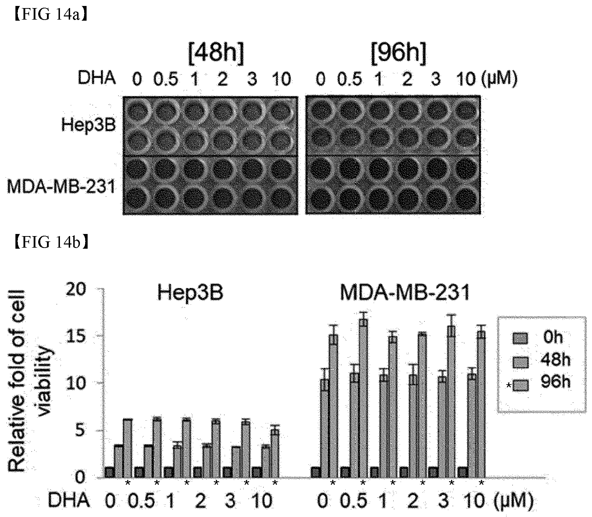

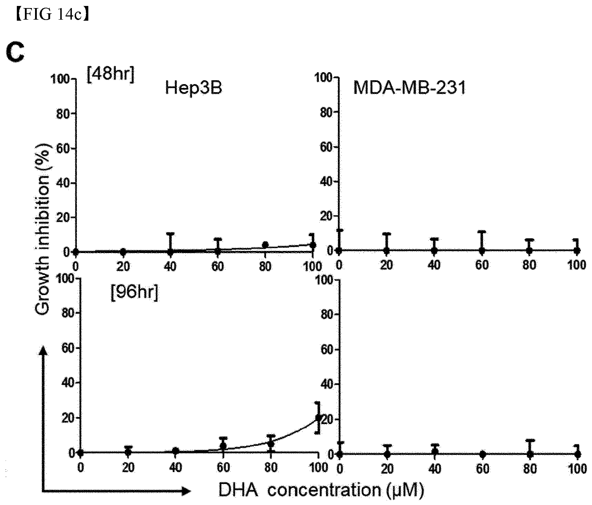

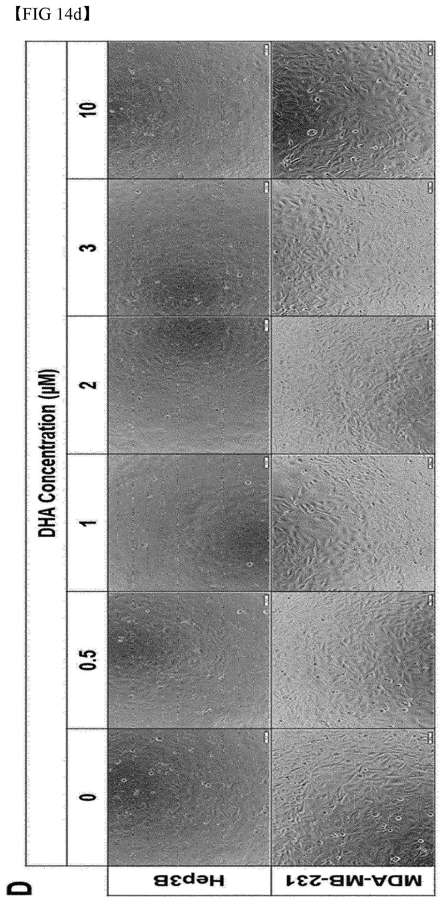

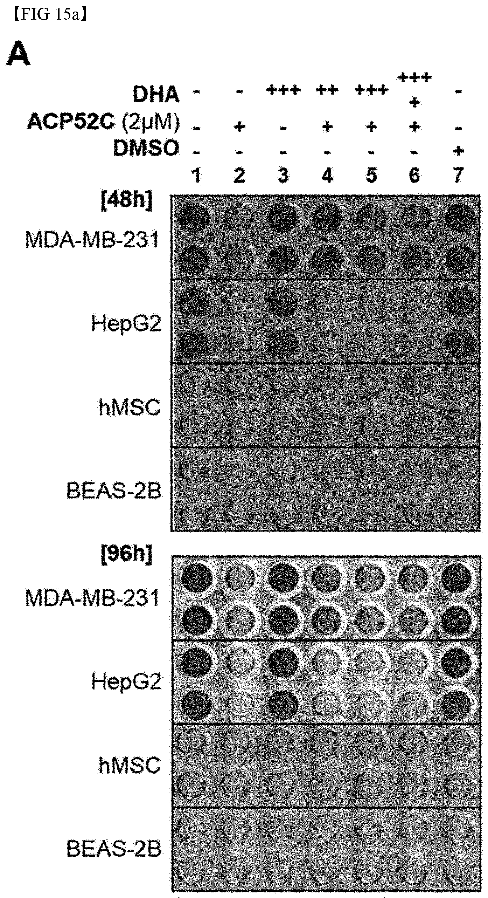

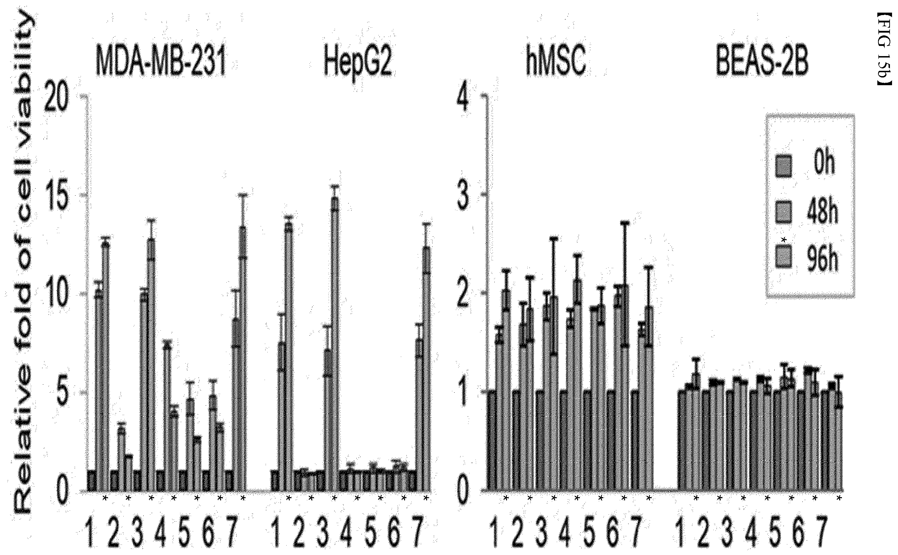

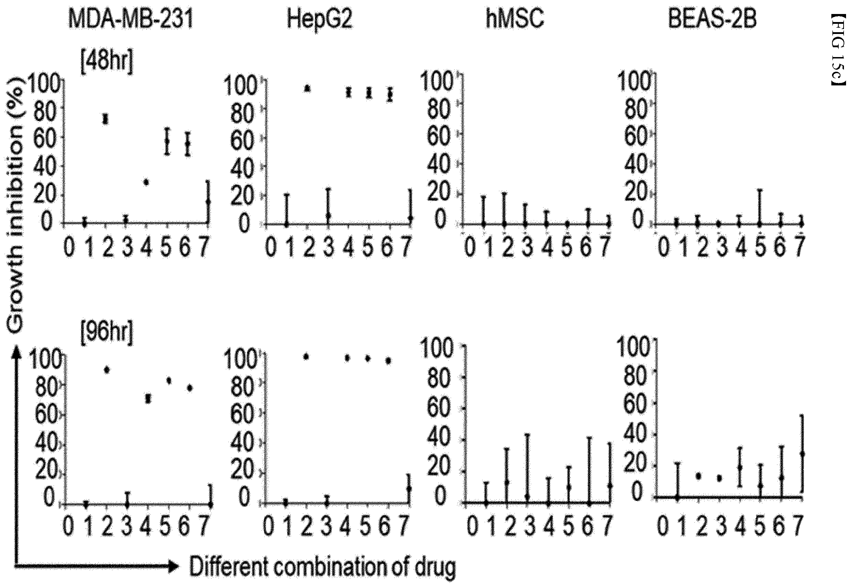

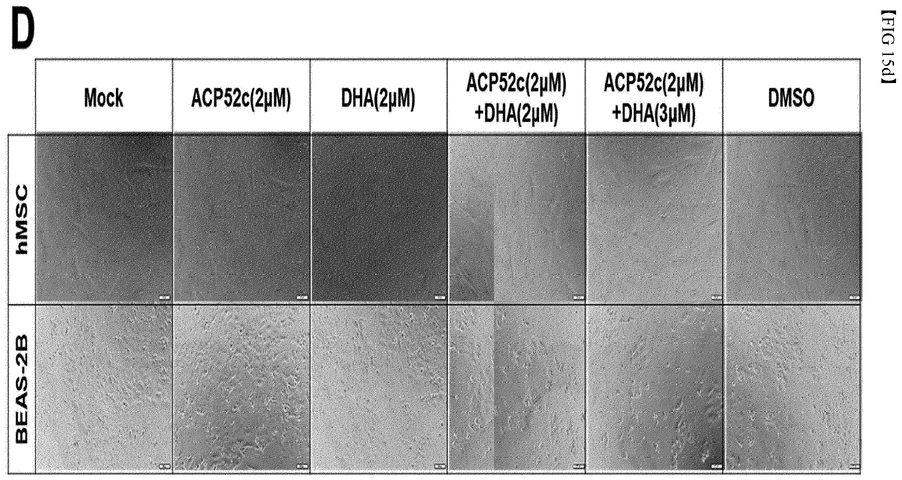

First, to test the effect of DHA itself, a breast cancer cell line (MDA-MB-231), liver cancer cell lines (Hep3B and HepG2), a normal hMSC cell line (LA211), and a non-tumorigenic lung cell line (BEAS-2B) were treated with DHA over concentrations, and then cell growth was analyzed by MTT assay. As a result, it showed no cytotoxicity on the normal cell line, but showed cytotoxicity at concentrations of 20 µM or more in cancer cells and BEAS-2B (FIG. 13). However, since ACP52C induces cancer cell-specific cell death at the effective concentration of 10 µM or less, it was analyzed whether DHA itself exhibits an anticancer effect at a concentration of 10 µM or less when DHA is used in combination with ACP52C. However, no anticancer effect was exhibited in Hep3B and MDA-MB-231 cell lines (FIG. 14). Meanwhile, when analyzed the cell death effect of 2 µM ACP52C in combination with 1 to 3 µM DHA, the combined treatment did not show any inhibitory or beneficial effect in the normal hMSC cell line (LA211) and the non-tumorigenic lung cell line (BEAS-2B), but showed an inhibitory effect in the breast cancer cell line (MDA-MB-231), exhibiting a lower efficacy than that of ACP52C alone (FIG. 15). Therefore, it was presumed that DHA can exhibit an anticancer effect at high concentrations, but DHA itself does not have an anticancer effect at a concentration for securing the ACP52C stability in vivo.

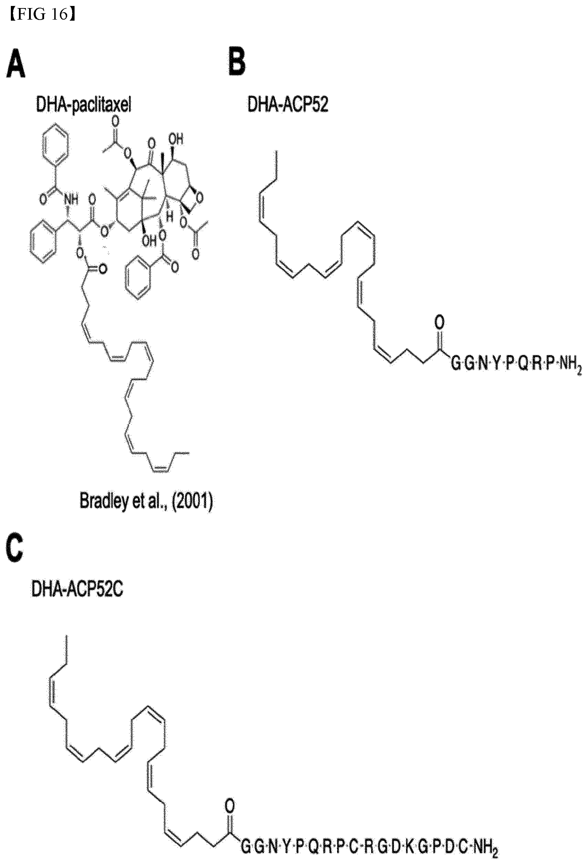

Accordingly, two peptides were synthesized, where DHA was directly conjugated to the N-terminus of ACP52: DHA-ACP52 (only DHA was conjugated to ACP52 since DHA has cell membrane permeability by itself) and DHA-ACP52C (both DHA and a cell-penetrating peptide iRGD were conjugated) (FIG. 16).

### [Example 4] Confirmation of CP2c-targeting peptide-DHA conjugates on the in vitro anticancer effect

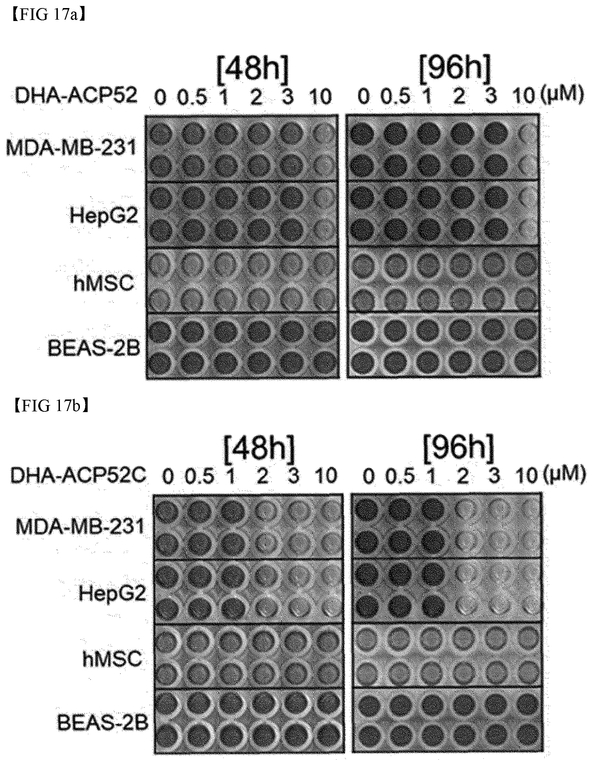

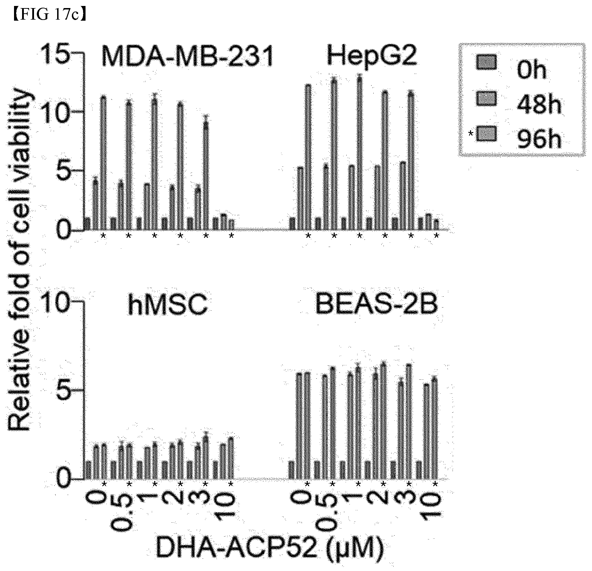

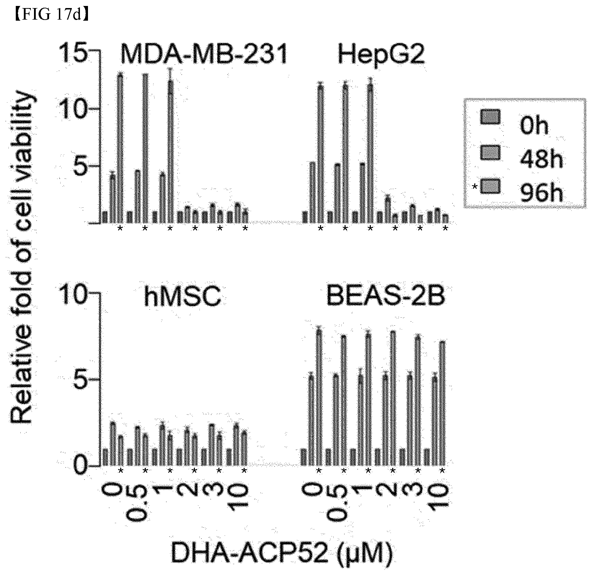

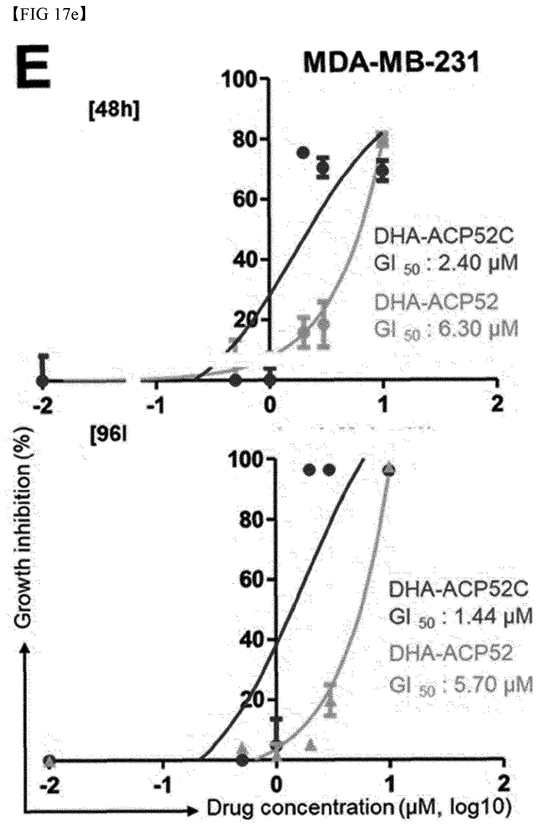

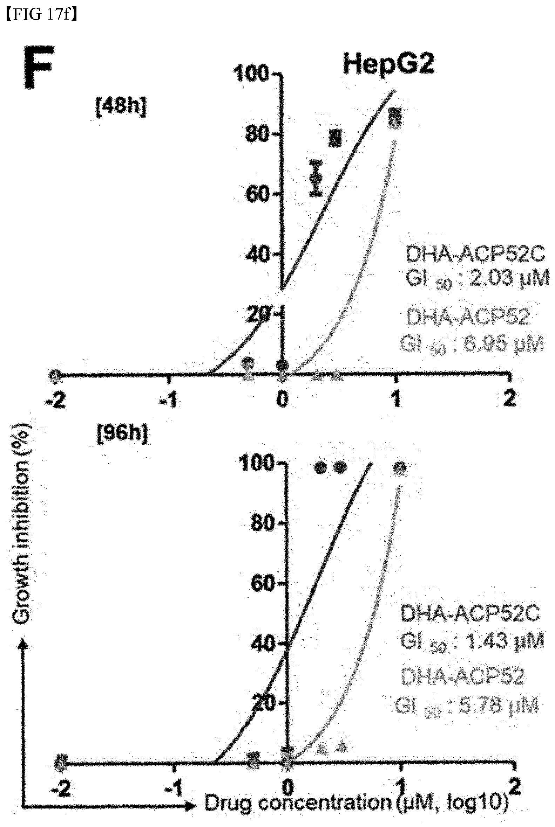

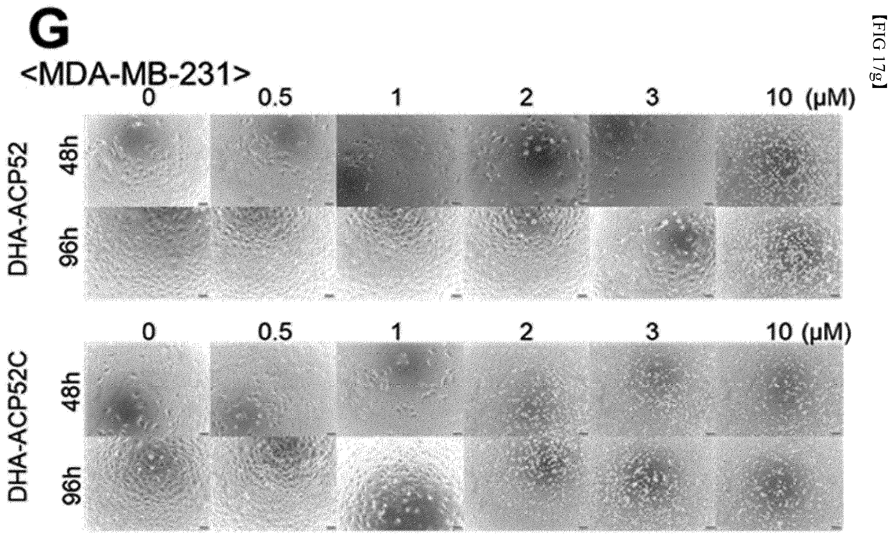

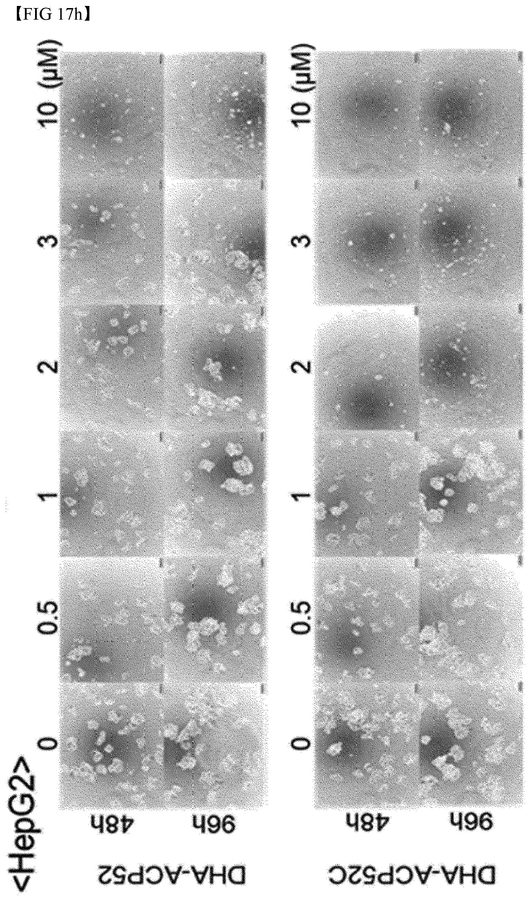

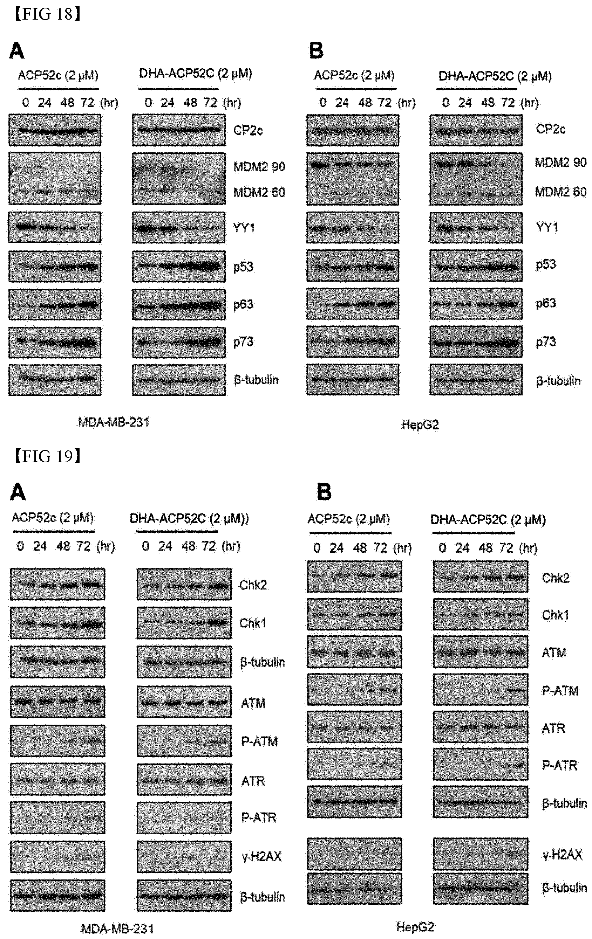

When with DHA-binding peptides were treated to a normal cell line and a cancer cell line and then cell growth was monitored by MTT assay, none of DHA-ACP52 and DHA-ACP52C showed toxicity in the normal cell line and the non-tumorigenic BEAS-2B cell line. In breast cancer (MDA-MB-231) and liver cancer (HepG2) cell lines, DHA-ACP52 showed the GI₅₀ value of 5.7 to 6.95 µM, indicating this efficacy was rather inferior to 2 µM of ACP52C, whereas DHA-ACP52C showed the GI₅₀ value of 1.43 to 2.4 µM, a similar efficacy to ACP52C (FIG. 17). In addition, DHA-ACP52C treatment showed similar expression profiles of factors involved in the intracellular apoptosis and cell cycle arrest to ACP52C treatment (FIGS. 18 and 19). Therefore, it was presumed that DHA has insignificant cell permeability compared to iRGD since the anticancer effect of DHA-ACP52 is inferior to ACP52C, although it is known that DHA itself enters the cell through the cell membrane.

### [Example 5] Tests of CP2c-targeting peptide-DHA conjugates on tumor growth inhibition and general physiological toxicity in various cancer cell line xenograft mouse models

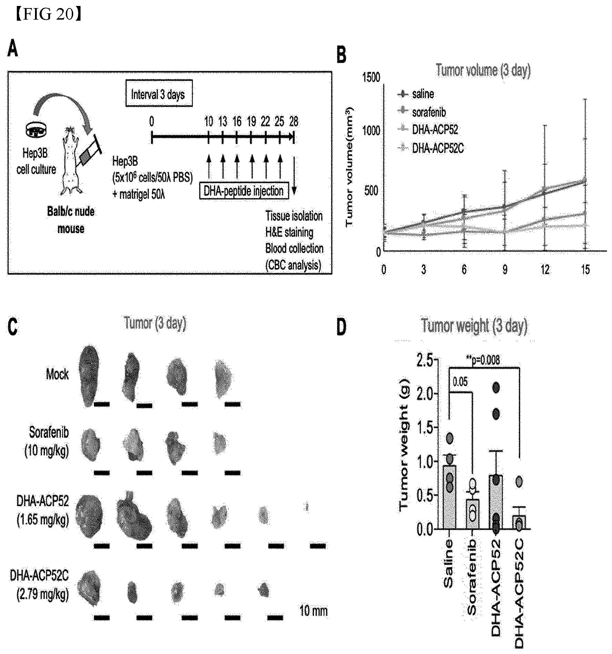

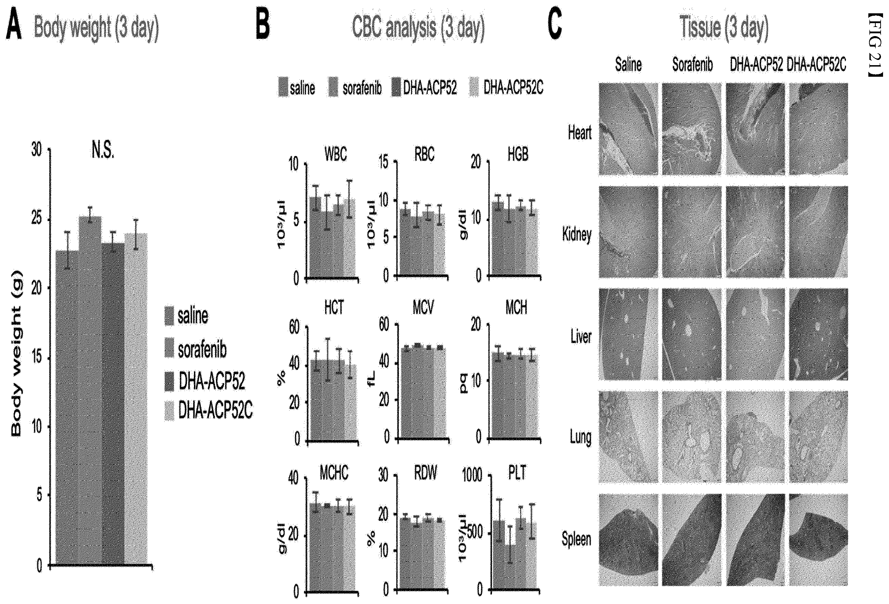

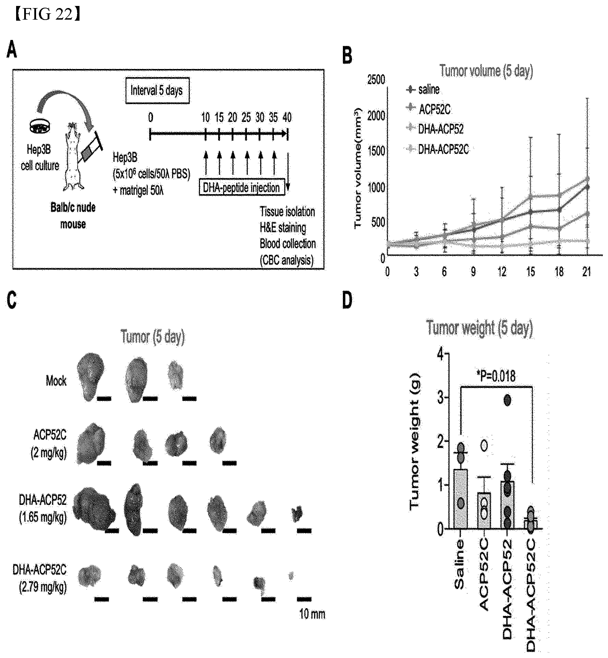

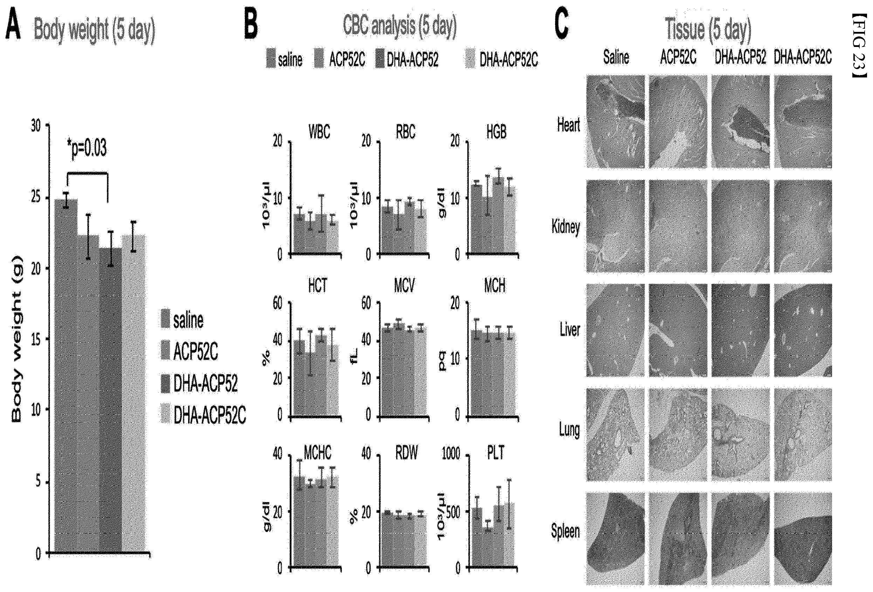

The efficacy of the CP2c-targeting peptide-DHA conjugates that was verified at the cell line level was also confirmed *in vivo* in the Hep3B xenograft mouse model. Along with saline (mock) as a negative control and sorafenib (10 mg/kg) as a positive control, DHA-ACP52 (1.65 mg/kg) and DHA-ACP52C (2.79 mg/kg) as experimental groups were injected into the Hep3B xenograft mice through the tail vein a total of 6 times at 3-day intervals (FIG. 20A). When measured the tumor size of mice once every 3 days during the experiment, it was confirmed that the groups treated with sorafenib and DHA-ACP52C inhibited cancer growth more effectively than the group treated with mock or DHA-ACP52 (FIG. 20B). When analyze the tumor size, shape, and weight in the sacrificed mice after the completion of experiment, DHA-ACP52C exhibited better anticancer efficacy than sorafenib although sorafenib exhibited significant anticancer efficacy compared to the mock, while DHA-ACP52 did not exhibit any effect (FIGS. 20C and 20D). Considering that DHA-ACP52C-treated mice showed similar body weight, hematological and histological parameters to different treatment groups including the control, it could be presumed that DHA-ACP52C would not exhibit any adverse side effect on normal cells *in vivo* (FIG. 21). On the other hand, when an anticancer effect of DHA-ACP52 (1.65 mg/kg) and DHA-ACP52C (2.79 mg/kg) in vivo was analyzed in the Hep3B xenograft mouse model by tail vein injection total of 6 times at 5-day intervals, along with groups of a negative control saline (mock) and a positive control ACP52C(2 mg/kg) (FIG. 22A), and measured the tumor size of mice every 3 days during the experiment, DHA-ACP52C showed better efficacy than ACP52C while DHA-ACP52 showed a similar efficacy to the negative control (FIG. 22B). When analyzed the tumor size, shape, and weight in the sacrificed mice after the completion of experiment, only DHA-ACP52C exhibited a significant anticancer efficacy (FIGS. 22C and 22D). Considering that DHA-ACP52C-treated mice showed similar body weight, hematological and histological parameters to different treatment groups including the control, it could be presumed that DHA-ACP52C would not exhibit any adverse side effect on normal cells *in vivo* (FIG. 23).

As a summary, DHA-ACP52C exhibited a cancer cell growth inhibitory effect similar to that of conventional ACP52C at the cell line level, but DHA-ACP52C exhibited a superior anticancer effect compared to ACP52C in the Hep3B xenograft mouse model. In particular, ACP52C exhibited a significant anticancer effect when injected by 3-day intervals, but did not exhibit a significant anticancer effect when injected by 5-day intervals (FIGS. 11 and 20). On the contrary, DHA-ACP52C exhibited a clear anticancer effect when injected by 3- or 5-day intervals (FIGS. 20 and 22). Therefore, it was confirmed that DHA improves the in vivo stability of ACP52C in DHA-ACP52C, but does not exert any adverse side effect in normal cells and normal tissues.

## Claims

1. A CP2c-targeting peptide-unsaturated fatty acid conjugate comprising: a transcription factor CP2c-targeting peptide consisting of 4 to 20 amino acids, and
an unsaturated fatty acid conjugated to the targeting peptide,
wherein the transcription factor CP2c-targeting peptide comprises an amino acid sequence represented by SEQ ID NO: 1 or 2.

2. The CP2c-targeting peptide-unsaturated fatty acid conjugate of claim 1, further comprising a cell-penetrating peptide (CPP) binding to the C-terminus of the CP2c-targeting peptide and represented by SEQ ID NO: 3.

3. The CP2c-targeting peptide-unsaturated fatty acid conjugate of claim 1, wherein the peptide C-terminus of the CP2c-targeting peptide-unsaturated fatty acid conjugate is modified in order to enhance stability.

4. The CP2c-targeting peptide-unsaturated fatty acid conjugate of claim 3, wherein the peptide C-terminus of the CP2c-targeting peptide-unsaturated fatty acid conjugate is modified with an amide group.

5. The CP2c-targeting peptide-unsaturated fatty acid conjugate of claim 1, further comprising a linker peptide bound to the N-terminus of the CP2c-targeting peptide, wherein the linker peptide consists of an amino acid sequence represented by Gₙ and n is an integer from 0 to 6.

6. The CP2c-targeting peptide-unsaturated fatty acid conjugate of claim 1, wherein the unsaturated fatty acid is all-cis-docosa-4,7,10,13,16,19-hexa-enoic acid (DHA).

7. The CP2c-targeting peptide-unsaturated fatty acid conjugate of claim 5, wherein a carboxyl group of the unsaturated fatty acid binds to an amino group of the linker peptide.

8. A pharmaceutical composition for preventing or treating cancer, comprising the CP2c-targeting peptide-unsaturated fatty acid conjugate of any one of claims 1 to 7 as an active ingredient.

9. A health functional food composition for preventing or ameliorating cancer, comprising the CP2c-targeting peptide-unsaturated fatty acid conjugate of any one of claims 1 to 7 as an active ingredient.

10. A use of the CP2c-targeting peptide-unsaturated fatty acid conjugate of any one of claims 1 to 7 for preventing or treating cancer.

11. A method for preventing or treating cancer, the method comprising: administering the composition of claim 8 to an individual.
